# EUROPEAN PATENT APPLICATION

(11) **EP 4 524 149 A1**
(43) Date of publication of application: **19.03.2025**
(21) Application number: 23802912.8
(22) Date of filing: 09.05.2023
(51) Int. Cl.: C07K 7/06, C07K 14/725, C12N 15/12, C12N 15/867, C12N 5/10, A61K 39/00, A61P 35/00, A61P 31/20

(54) **USE OF ANTIGEN SHORT PEPTIDE IN SCREENING DRUG FOR TREATING HPV-RELATED DISEASES AND TCR SCREENED BY SAME**

(30) Priority: 10.05.2022 CN 202210503002
(71) Applicant: GUANGZHOU MEDICAL UNIVERSITY, Guangzhou, Guangdong 511436 (CN)
(72) Inventor: CHEN, Lin, Guangzhou, Guangdong 511436 (CN); WANG, Xiaoling, Guangzhou, Guangdong 511436 (CN)
(74) Representative: Lavoix
(86) International application number: PCT/CN2023/093015
(87) International publication number: WO 2023/217143

(57) **Abstract**

Disclosed in the present application are the use of an antigen short peptide in screening a drug for treating HPV-related diseases and a TCR screened by same, wherein the amino acid sequence of the antigen short peptide is as shown in SEQ ID NO: 1 or SEQ ID NO: 2. In the present application, the antigen short peptide can be used to screen a specific T cell receptor (TCR), and the T cell transduced with the TCR can be specifically activated and have a very strong killing effect on tumor cells expressing A1101 and HPV, which can be used for immunotherapy of HPV positive tumors, such as cervical cancer. In addition, the T cell transduced with the TCR of the present application has a strong activation reaction on a cell line expressing E6, has no activation reaction on a cell line not expressing E6, and has a very strong killing function on the cell line expressing E6 and can effectively inhibit the growth of E6 positive tumors.

## Description

### CROSS-REFERENCE TO RELATED APPLICATIONS

This application claims priority to Chinese patent application No. CN202210503002.7 filed on May 10, 2022, the disclosure of which is incorporated herein by reference in its entirety.

### REFERENCE TO ELECTRONIC SEQUENCE LISTING

The content of the electronic sequence listing (TFG00782PCT-XLB.xml; size: 52,426 bytes; creation date: May 6, 2023) are incorporated herein by reference in its entirety.

### TECHNICAL FIELD

The present application relates to the field of medical technology, and in particular to the use of an antigenic short peptide in screening drugs for treating HPV-related diseases and the TCR screened therefrom.

### BACKGROUND

Human papillomavirus (HPV) is a DNA virus belonging to the genus Papillomavirus in the family Papillomaviridae. This kind of virus infects human epidermis and mucosal tissues. Currently, about 170 kinds of HPV have been identified. Sometimes HPV can cause warts or even cancer after invading human body, but most of the time there is no any clinical symptom.

An important factor in cancer caused by HPV is persistent infection of high-risk HPV, such as HPV16, HPV18, etc. The E6 and E7 proteins encoded by high-risk HPV can inhibit the activity of tumor suppressor genes p53 and Rb proteins, respectively, thereby leading to cell cycle abnormalities and carcinogenesis. High-risk HPVs are associated with 90% of cervical and anal cancers, 40% to 60% of vaginal and penile cancers, and may also be associated with 60% of oropharyngeal cancers, depending on geographical characteristics.

Cervical cancer is the most common malignant tumor of the female reproductive tract, and its global incidence rate ranks second among female malignant tumors. The incidence rate of cervical cancer in developing countries is significantly higher than that in developed countries. According to the latest statistics in 2018, the incidence rate of cervical cancer in China is 15.30/100,000 and the mortality rate is 4.57/100,000, which are higher than the global average incidence rate (10.61/100,000) and mortality rate (2.98/100,000). In the past 10 years, the incidence rate of cervical cancer has been on the rise, with the peak age of onset being 40-60 years old. Although surgery and chemoradiotherapy are effective in treating early-stage cervical cancer, more than half of patients are diagnosed with locally advanced cervical cancer at their first visit. 30%-70% of patients with locally advanced cervical cancer will experience recurrence and/or distant metastasis. For locally advanced and metastatic cervical cancer, the efficacy of traditional treatment is very limited, with a five-year survival rate of only 57.1% and 17.3%.

Specific T cell immunotherapy refers to the method of using specific T cells targeting tumor antigens to kill tumor cells. It is a highly personalized tumor immunotherapy method. Due to the existence of the tumor local immunosuppressive microenvironment, the autologous T cells in patients have limited ability to kill tumors. Therefore, people try to improve the ability of T cells to kill tumors by genetically modifying them. Both TCR-T and CAR-T are genetically modified cell therapy drugs. After the transferred T cell receptor (TCR) or chimeric antigen receptor (CAR) gene is bound to their respective targets, T cells can be activated. The tumor cells are eliminated by using granzymes, perforin, cytokines, and other substances released by T cells; but the significant difference between TCR-T and CAR-T is that the target of CAR-T is the membrane protein on the cell surface, while the target of TCR-T is the antigenic short peptide-MHC complex (peptide-major histocompatibility complex, pMHC).

HPV-related proteins are the most ideal T cell immunotherapy targets for cervical cancer. The target recognized by TCR is the "antigenic short peptide-MHC molecule complex". TCR has MHC-restriction at the same time. In theory, a TCR molecule can only specifically recognize a short peptide presented by a specific MHC. MHC is polymorphic. Currently, more than 15,000 human MHC (also known as human leukocyte antigen, HLA) alleles have been discovered, and the frequency of occurrence of specific HLA varies greatly in different populations. The most common HLA type in the Chinese population is HLA-A1101. The short peptides presented by HLA class I molecules are 8-11 amino acids in length, and the short peptides presented by HLA class II molecules are 12-24 amino acids in length. The discovery and identification of these antigenic short peptides is a prerequisite for TCR-T therapy. Although whether a short peptide binds to HLA can be determined through affinity prediction, HLA binding assay, etc., whether the short peptide can be naturally presented by HPV-expressing tumor cells is the key to determining whether the short peptide-specific TCR can be used for tumor treatment.

Therefore, those skilled in the art are devoted to finding A1101-restricted HPV antigenic short peptides, and using the discovered antigenic short peptides to screen TCRs that can specifically recognize HPV-positive tumor cells, so that they can play a role in T cell immunotherapy.

### SUMMARY

The purpose of the present application is to provide a use of an antigenic short peptide in screening drugs for treating HPV-related diseases, as well as the T cell receptor (TCR) screened thereby, wherein the antigenic short peptide is used to screen specific T cell antibodies, and the T cells that transcribe the TCR can be specifically activated and have a strong killing effect on tumor cells expressing A1101 and HPV.

The specific technical solutions of the present application are as follows:
1. Use of an antigenic short peptide in screening drugs for treating HPV-related diseases, wherein the amino acid sequence of the antigenic short peptide is represented by SEQ ID NO: 1 or SEQ ID NO: 2.
2. The use according to item 1, wherein the HPV-related disease is chronic HPV infection, cervical intraepithelial neoplasia, cervical cancer, head and neck cancer, anal cancer, penile cancer, vaginal cancer or vulvar cancer.
3. Use of an antigenic short peptide in screening drugs for treating chronic HPV infection, cervical intraepithelial neoplasia, cervical cancer, head and neck cancer, anal cancer, penile cancer, vaginal cancer, or vulvar cancer, wherein the amino acid sequence of the antigenic short peptide is represented by SEQ ID NO: 1 or SEQ ID NO: 2.
4. The use according to any one of items 1-3, wherein the drug is a T cell receptor (TCR) for binding to an antigenic short peptide-HLA-A1101 complex comprising the antigenic short peptide.
5. A T cell receptor (TCR), wherein the TCR comprises an α chain comprising a variable region and/or a β chain comprising a variable region, and the variable region of the α chain comprises a complementarity determining region 1 (CDR1) having an amino acid sequence of SEQ ID NO: 3 or SEQ ID NO: 9; and/or
   a complementarity determining region 2 (CDR2) having an amino acid sequence of SEQ ID NO:4 or SEQ ID NO:10.
6. The T cell receptor (TCR) according to item 5, wherein the variable region of the β chain comprises:
   a complementarity determining region 1 (CDR1) having an amino acid sequence of SEQ ID NO: 6; and/or
   a complementarity determining region 2 (CDR2) having an amino acid sequence of SEQ ID NO:7.
7. The T cell receptor (TCR) according to item 5 or 6, wherein the variable region of the α chain comprises: a complementarity determining region 3 (CDR3) comprising an amino acid sequence of SEQ ID NO: 5, SEQ ID NO: 11, SEQ ID NO: 13 or SEQ ID NO: 15; and/or
   the variable region of the β chain comprises: a complementarity determining region 3 (CDR3) comprising an amino acid sequence of SEQ ID NO:8, SEQ ID NO:12, SEQ ID NO:14 or SEQ ID NO:16.
8. The T cell receptor (TCR) according to any one of items 5-7, wherein the variable region of the α chain further comprises a first leader sequence; and/or
   the variable region of the β chain further comprises a second leader sequence.
9. The T cell receptor (TCR) according to any one of items 5-8, wherein the amino acid sequence of the α chain variable region is represented by SEQ ID NO: 20, SEQ ID NO: 22, SEQ ID NO: 24 or SEQ ID NO: 26, or is an amino acid sequence having at least 90% sequence identity with SEQ ID NO: 20, SEQ ID NO: 22, SEQ ID NO: 24 or SEQ ID NO: 26; and/or
   the amino acid sequence of the variable region of the β chain is represented by SEQ ID NO:21, SEQ ID NO:23, SEQ ID NO:25 or SEQ ID NO:27, or is an amino acid sequence having at least 90% sequence identity with SEQ ID NO:21, SEQ ID NO:23, SEQ ID NO:25 or SEQ ID NO:27.
10. The T cell receptor (TCR) according to any one of items 5-9, wherein the α chain further comprises an α constant region, and/or the β chain further comprises a β constant region; preferably, the constant region is a mouse constant region or a human constant region.
11. The T cell receptor (TCR) according to any one of items 5-10, wherein the TCR is isolated or purified, or is recombinant.
12. The T cell receptor (TCR) according to any one of items 5-11, wherein the TCR is human.
13. The T cell receptor (TCR) according to any one of items 5-12, wherein the TCR is monoclonal.
14. The T cell receptor (TCR) according to any one of items 5-13, wherein the TCR is a single chain.
15. The T cell receptor (TCR) according to any one of items 5-14, wherein the TCR comprises two chains.
16. The T cell receptor (TCR) according to any one of items 5-15, wherein the TCR is in a cell-bound form or in a soluble form, preferably in a soluble form.
17. The T cell receptor (TCR) according to any one of items 5-16, wherein the TCR binds to the antigenic short peptide-HLAA1101 complex, and preferably, the amino acid sequence of the antigenic short peptide is represented by SEQ ID NO: 1 or SEQ ID NO: 2.
18. A nucleic acid molecule, wherein the nucleic acid molecule comprises: an nucleotide sequence encoding the TCR or the α chain or β chain thereof according to any one of items 5-17.
19. The nucleic acid molecule according to item 18, wherein the nucleotide sequence encoding the α chain comprises a nucleotide sequence of SEQ ID NO: 30, SEQ ID NO: 32, SEQ ID NO: 34 or SEQ ID NO: 36; and/or
   the nucleotide sequence encoding the β chain comprises a nucleotide sequence of SEQ ID NO:31, SEQ ID NO:33, SEQ ID NO:35 or SEQ ID NO:37.
20. A vector, wherein the vector comprises the nucleic acid molecule according to item 18 or 19.
21. The vector according to item 20, wherein the vector is an expression vector.
22. The vector according to item 20 or 21, wherein the vector is a viral vector, preferably a retroviral vector.
23. The vector according to item 22, wherein the viral vector is a lentiviral vector.
24. An engineered cell, comprising: the TCR according to any one of items 5-17, the nucleic acid molecule according to any one of items 18-19, or the vector according to any one of items 20-23.
25. The engineered cell according to item 24, wherein the TCR is heterologous to the cell.
26. The engineered cell according to item 24 or 25, wherein the engineered cell is a cell line.
27. The engineered cell according to any one of items 24-26, wherein the engineered cell is a primary cell obtained from a subject, preferably, the subject is a mammalian subject, preferably a human.
28. The engineered cell according to any one of items 24-27, wherein the engineered cell is a T cell or a NK cell, preferably, the T cell is a T cell isolated from peripheral blood.
29. The engineered cell according to item 28, wherein the T cell is CD8+ or CD4+.
30. A method for producing the engineered cell according to any one of items 24-29, comprising introducing the nucleic acid molecule according to any one of items 18-19 or the vector according to any one of items 20-23 into a cell in vitro or ex vivo.
31. The method according to item 30, wherein the vector is a viral vector and the introduction is performed by transduction.
32. A pharmaceutical composition, comprising: the T cell receptor (TCR) according to any one of items 5-17, the nucleic acid molecule according to any one of items 18-19, the vector according to any one of items 20-23, or the engineered cell according to any one of items 24-29.
33. The pharmaceutical composition according to item 32, further comprising a pharmaceutically acceptable carrier or adjuvant.
34. Use of the T cell receptor (TCR) according to any one of items 5-17, the nucleic acid molecule according to any one of items 18-19, the vector according to any one of items 20-23, the engineered cell according to any one of items 24-29, or the pharmaceutical composition according to any one of items 32-33 in the preparation of a medicament for treating HPV-related diseases.
35. The use according to item 34, wherein the HPV-related disease is chronic HPV infection, cervical intraepithelial neoplasia, cervical cancer, head and neck cancer, anal cancer, penile cancer, vaginal cancer or vulvar cancer.
36. A method for treating HPV-related diseases, comprising administering the T cell receptor (TCR) according to any one of items 5-17, the nucleic acid molecule according to any one of items 18-19, the vector according to any one of items 20-23, the engineered cell according to any one of items 24-29, or the pharmaceutical composition according to any one of items 32-33 to a subject in need thereof.
37. The method according to item 36, wherein the HPV-related disease is chronic HPV infection, cervical intraepithelial neoplasia, cervical cancer, head and neck cancer, anal cancer, penile cancer, vaginal cancer or vulvar cancer.

### EFFECTS OF THE INVENTION

The antigenic short peptide of the present application can be used to screen specific T cell receptors (TCRs), and the T cells transduced with such TCRs can be specifically activated and have a strong killing effect on tumor cells expressing A1101 and HPV, thus being used for the immunotherapy of HPV-positive tumors such as cervical cancer.

Furthermore, T cells transduced with the TCR described in the present application have strong activation responses to cell lines expressing E6, no activation response to cell lines not expressing E6, and have strong killing functions to cell lines expressing E6, and can effectively inhibit the growth of E6-positive tumors.

### BRIEF DESCRIPTION OF THE DRAWINGS

Fig. 1 shows the cloning of T cells specifically for the TTLEQQYNK and GTTLEQQYNK antigenic short peptides in Example 1, wherein Fig. 1A shows the flow cytometry results of T cells after two rounds of stimulation, and Fig.1B shows the flow cytometry results of T cells after three rounds of stimulation.
Fig. 2 shows the positive rate of transduction identified using flow cytometry in Example 3.
Fig. 3 shows the expression of NFAT in T cells transduced with the TCR described in Example 3 of the present application in response to target cells loaded with the TTLEQQYNK antigenic short peptide.
Fig. 4 shows the positive rate of transduction identified using flow cytometry in Example 4.
Fig. 5 shows the cell index for cell proliferation with T cells transduced with the TCR described in Example 4 of the present application.
Fig. 6 shows the tumor volume after transplanting T cells transduced with the TCR described in the present application in Example 5, wherein Fig. 6A shows the changes in tumor volume over time after transplanting the T cells, and Fig. 6B is a bar chart of tumor weight after transplanting T cells comprising different TCRs.
Figs. 7A and 7B show the observed growth status of organoids and the statistical diagram of integrity and incompleteness of organoids; wherein Fig. 7A shows the growth status of organoids observed under a microscope, and Fig. 7B is a statistical diagram of the number of organoids with intact and incomplete morphology.

### DETAILED DESCRIPTION

The present application is described in detail below in conjunction with the embodiments described in the drawings, wherein the same numbers in all the drawings represent the same features. Although specific embodiments of the present application are shown in the drawings, it should be understood that the present application can be implemented in various forms and should not be limited by the embodiments set forth herein. Instead, these embodiments are provided to enable a more thorough understanding of the present application and to fully convey the scope of the present application to those skilled in the art.

It should be noted that certain words used in the specification and claims refer to specific components. Those skilled in the art should understand that technicians may use different nouns to refer to the same component. This specification and the claims do not take the difference of nouns as a way to distinguish components, but take the difference of components in function as the criterion to distinguish them. "Comprising" or "including" as used throughout the specification and claims are open-ended and should be interpreted as "comprising, but not limited to." The following descriptions are preferred embodiments for implementing the present application, however, the description is intended to illustrate general principles of the specification and is not intended to limit the scope of the application. The protection scope of the present application shall be defined by the appended claims.

The present application provides the use of an antigenic short peptide in screening drugs for treating HPV-related diseases, wherein the amino acid sequence of the antigenic short peptide is represented by SEQ ID NO: 1 or SEQ ID NO: 2.

The sequence of SEQ ID NO: 1 is TTLEQQYNK.

The sequence of SEQ ID NO: 2 is GTTLEQQYNK.

In one embodiment, the HPV-related disease is chronic HPV infection, cervical intraepithelial neoplasia, cervical cancer, head and neck cancer, anal cancer, penile cancer, vaginal cancer or vulvar cancer, etc.

The present application provides the use of an antigenic short peptide in screening drugs for treating chronic HPV infection, cervical intraepithelial neoplasia, cervical cancer, head and neck cancer, anal cancer, penile cancer, vaginal cancer or vulvar cancer, and the amino acid sequence of the antigenic short peptide is represented by SEQ ID NO: 1 or SEQ ID NO: 2.

In one embodiment, the drug is a T cell receptor (TCR) for binding to an antigenic short peptide-HLA-A1101 complex comprising the antigenic short peptide.

That is, the T cell receptor is obtained by screening with the antigenic short peptide, and the T cell receptor (TCR) binds to the TTLEQQYNK-HLA-A1101 complex or to the GTTLEQQYNK-HLA-A1101 complex.

The T cell receptor or TCR is the only receptor for specific antigenic peptides presented on the major histocompatibility complex (MHC). In the immune system, the direct physical contact between T cells and antigen-presenting cells (APCs) is triggered by the binding of the antigen-specific TCR to the pMHC complex, then other cell surface molecules of both T cells and APCs interact, which causes a series of subsequent cell signaling and other physiological reactions, thereby T cells with different antigen specificities exert immune effects on target cells.

The TCR is a molecule comprising a variable α and β chains or a variable γ and δ chains, and the molecule is able to bind specifically to a peptide bound to an MHC molecule. In some embodiments, the TCR is in the αβ form. In general, TCRs that exist in αβ and γδ forms are generally similar in structure, but the T cells expressing them can have different anatomical locations or functions, and TCRs can be found on cell surface or in soluble form. Typically, TCRs are found on the surface of T cells (T lymphocytes), where it is usually responsible for recognizing antigens bound to major histocompatibility complex (MHC) molecules.

The variable domain of a TCR comprises complementarity determining regions (CDRs), which are usually the main contributors to antigen recognition, binding ability and specificity for peptides, MHC and/or MHC-peptide complexes. The CDRs of a TCR or a combination thereof form all or substantially all of the antigen binding sites of a given TCR molecule. Individual CDRs within the variable region of a TCR are usually separated by framework regions (FRs). Among them, CDR3 is the main CDR responsible for antigen binding or specificity, or is the most important among the three CDRs on a given TCR variable region for antigen recognition and/or for interaction with the processed peptide portion of the peptide-MHC complex. In some cases, CDR1 of the α chain can interact with the N-terminal portion of certain antigenic peptides; in some cases, CDR1 of the β chain can interact with the C-terminal portion of certain antigenic peptides; in some cases, CDR2 has the strongest effect on the interaction with or recognition of the MHC portion of a MHC-peptide complex or is the main responsible CDR; in some cases, the variable region of the β chain comprises other hypervariable regions (CDR4 or HVR4), which are usually involved in superantigen binding rather than antigen recognition.

The TTLEQQYNK-HLA-A1101 complex or GTTLEQQYNK-HLA-A1101 complex refers to a complex formed by binding of HLA-A1101 and the antigenic short peptide TTLEQQYNK or GTTLEQQYNK. The protein is degraded into polypeptides of different lengths by proteases in cells, and a portion of the polypeptides bind to HLA to form a complex that is presented to the cell surface. The TTLEQQYNK-HLA-A1101 complex or GTTLEQQYNK-HLA-A1101 complex recognized by the TCR may be expressed on the cell membrane or exist in the solution in the form of a soluble protein.

The amino acid sequence of HLA-A1101 is represented by SEQ ID NO: 38, and its amino acid sequence is:

MAVMAPRTLLLLLSGALALTQTWAGSHSMRYFYTSVSRPGRGEPRFIAVG
YVDDTQFVRFDSDAASQRMEPRAPWIEQEGPEYWDQETRNVKAQSQTDRVD
LGTLRGYYNQSEDGSHTIQIMYGCDVGPDGRFLRGYRQDAYDGKDYIALNED
LRSWTAADMAAQITKRKWEAAHAAEQQRAYLEGRCVEWLRRYLENGKETL
QRTDPPKTHMTHHPISDHEATLRCWALGFYPAEITLTWQRDGEDQTQDTELVE
TRPAGDGTFQKWAAVVVPSGEEQRYTCHVQHEGLPKPLTLRWELSSQPTIPIV
GIIAGLVLLGAVITGAVVAAVMWRRKSSDRKGGSYTQAASSDSAQGSDVSLTA
CKVSR.

In one embodiment, the antigenic short peptide is used for use in screening drugs for treating HPV-related diseases, and the amino acid sequence of the antigenic short peptide is represented by SEQ ID NO: 1 or SEQ ID NO: 2. In one embodiment, the HPV-related disease is chronic HPV infection, cervical intraepithelial neoplasia, cervical cancer, head and neck cancer, anal cancer, penile cancer, vaginal cancer or vulvar cancer. In one embodiment, the drug is a T cell receptor (TCR) for binding to an antigenic short peptide-HLA-A1101 complex comprising the antigenic short peptide.

In one embodiment, the antigenic short peptide is used for use in screening drugs for treating chronic HPV infection, cervical intraepithelial neoplasia, cervical cancer, head and neck cancer, anal cancer, penile cancer, vaginal cancer or vulvar cancer, and the amino acid sequence of the antigenic short peptide is represented by SEQ ID NO:1 or SEQ ID NO:2. In one embodiment, the drug is a T cell receptor (TCR) for binding to an antigenic short peptide-HLA-A1101 complex comprising the antigenic short peptide.

The present application provides a T cell receptor (TCR), wherein the TCR comprises an α chain comprising a variable region and/or a β chain comprising a variable region, and the variable region of the α chain comprises:
a complementary determining region 1 (CDR1) with an amino acid sequence of SEQ ID NO: 3 or SEQ ID NO: 9; and/or
a complementarity determining region 2 (CDR2) with an amino acid sequence of SEQ ID NO:4 or SEQ ID NO:10.
The amino acid sequence of SEQ ID NO:3 is: SSYSPS;
The amino acid sequence of SEQ ID NO:9 is: TTLSN;
The amino acid sequence of SEQ ID NO:4 is: YTSAATLV;
The amino acid sequence of SEQ ID NO: 10 is: LVKSGEV.

In one embodiment, the variable region of the β chain comprises:
a complementarity determining region 1 (CDR1) with an amino acid sequence of SEQ ID NO: 6; and/or
a complementarity determining region 2 (CDR2) with an amino acid sequence of SEQ ID NO:7.
The amino acid sequence of SEQ ID NO:6 is: SGDLS;
The amino acid sequence of SEQ ID NO:7 is: YYNGEE.

In one embodiment, the variable region of the α chain comprises: a complementarity determining region 3 (CDR3) with an amino acid sequence of SEQ ID NO: 5, SEQ ID NO: 11, SEQ ID NO: 13 or SEQ ID NO: 15; and/or
the variable region of the β chain comprises: a complementarity determining region 3 (CDR3) with an amino acid sequence of SEQ ID NO:8, SEQ ID NO: 12, SEQ ID NO:14 or SEQ ID NO:16.
The amino acid sequence of SEQ ID NO:5 is: VVSLSGGYNKLI;
The amino acid sequence of SEQ ID NO: 11 is: AGPKITGGGNKLT;
The amino acid sequence of SEQ ID NO: 13 is: AGPILTGGGNKLT;
The amino acid sequence of SEQ ID NO: 15 is: AGPVLTGGGNKLT;
The amino acid sequence of SEQ ID NO:8 is: ASSVTGSGYT;
The amino acid sequence of SEQ ID NO: 12 is: ASSVGGGPNYGYT;
The amino acid sequence of SEQ ID NO: 14 is: ASGLSGPNTGELF;
The amino acid sequence of SEQ ID NO: 16 is: ASSVGGPNTGELF.

In one embodiment, the variable region of the α chain further comprises a first leader sequence; and/or
the variable region of the β chain further comprises a second leader sequence.

The first leader sequence of the variable region of the α chain and the second leader sequence of the variable region of the β chain are well known to those skilled in the art. For example, the first leader sequence of the variable region of the α chain can use a leader sequence with an amino acid sequence of SEQ ID NO: 17 or SEQ ID NO: 19, and the second leader sequence of the variable region of the β chain can use a leader sequence with an amino acid sequence of SEQ ID NO: 18.
The amino acid sequence of SEQ ID NO: 17 is: MLLLLVPVLEVIFTLGGTR;
The amino acid sequence of SEQ ID NO: 19 is: MLLITSMLVLWMQLSQVN;
The amino acid sequence of SEQ ID NO: 18 is: MGFRLLCCVAFCLLGAGPV.

In one embodiment, the amino acid sequence of the variable region of the α chain is represented by SEQ ID NO:20, SEQ ID NO:22, SEQ ID NO:24 or SEQ ID NO:26, or is an amino acid sequence having at least 90% sequence identity with SEQ ID NO:20, SEQ ID NO:22, SEQ ID NO:24 or SEQ ID NO:26; and/or
the amino acid sequence of the variable region of the β chain is represented by SEQ ID NO:21, SEQ ID NO:23, SEQ ID NO:25 or SEQ ID NO:27, or is an amino acid sequence having at least 90% sequence identity with SEQ ID NO:21, SEQ ID NO:23, SEQ ID NO:25 or SEQ ID NO:27.
The amino acid sequence of SEQ ID NO:20 is: 
MLLLLVPVLEVIFTLGGTRAQSVTQLDSHVSVSEGTPVLLRCNYSSSYSPS
LFWYVQHPNKGLQLLLKYTSAATLVKGINGFEAEFKKSETSFHLTKPSAHMSD
AAEYFCVVSLSGGYNKLIFGAGTRLAVHPY.
The amino acid sequence of SEQ ID NO:21 is: 
MGFRLLCCVAFCLLGAGPVDSGVTQTPKHLITATGQRVTLRCSPRSGDLS
VYWYQQSLDQGLQFLIQYYNGEERAKGNILERFSAQQFPDLHSELNLSSLELG
DSALYFCASSVTGSGYTFGSGTRLTVV.
The amino acid sequence of SEQ ID NO:22 is: 
MLLITSMLVLWMQLSQVNGQQVMQIPQYQHVQEGEDFTTYCNSSTTLS
NIQWYKQRPGGHPVFLIQLVKSGEVKKQKRLTFQFGEAKKNSSLHITATQTTD
VGTYFCAGPKITGGGNKLTFGTGTQLKVELN.
The amino acid sequence of SEQ ID NO:23 is: 
MGFRLLCCVAFCLLGAGPVDSGVTQTPKHLITATGQRVTLRCSPRSGDLS
VYWYQQSLDQGLQFLIQYYNGEERAKGNILERFSAQQFPDLHSELNLSSLELG
DSALYFCASSVGGGPNYGYTFGSGTRLTVV.
The amino acid sequence of SEQ ID NO:24 is: 
MLLITSMLVLWMQLSQVNGQQVMQIPQYQHVQEGEDFTTYCNSSTTLS
NIQWYKQRPGGHPVFLIQLVKSGEVKKQKRLTFQFGEAKKNSSLHITATQTTD
VGTYFCAGPILTGGGNKLTFGTGTQLKVELN.
The amino acid sequence of SEQ ID NO:25 is: 
MGFRLLCCVAFCLLGAGPVDSGVTQTPKHLITATGQRVTLRCSPRSGDLS
VYWYQQSLDQGLQFLIQYYNGEERAKGNILERFSAQQFPDLHSELNLSSLELG
DSALYFCASGLSGPNTGELFFGEGSRLTVL.
The amino acid sequence of SEQ ID NO:26 is: 
MLLITSMLVLWMQLSQVNGQQVMQIPQYQHVQEGEDFTTYCNSSTTLS
NIQWYKQRPGGHPVFLIQLVKSGEVKKQKRLTFQFGEAKKNSSLHITATQLTD
VGTYFCAGPVLTGGGNKLTFGTGTQLKVELN.
The amino acid sequence of SEQ ID NO:27 is: 
MGFRLLCCVAFCLLGAGPVDSGVTQTPRYLITATGQRVTLRCSPRSGDLS
VYWYQQSLDQGLQFLIQYYNGEERAKGNILERFSAQQFPDLHSELNLSSLELG
DSALYFCASSVGGPNTGELFFGEGSRLTVL.
The amino acid sequence of the variable region of the α chain is an amino acid sequence having at least 90% sequence identity with SEQ ID NO:20, SEQ ID NO:22, SEQ ID NO:24 or SEQ ID NO:26, for example it can be an amino acid sequence having 90%, 91%, 92%, 93%, 94%, 95%, 96%, 97%, or 98% sequence identity with SEQ ID NO:20, SEQ ID NO:22, SEQ ID NO:24 or SEQ ID NO:26;
The amino acid sequence of the variable region of the β chain is an amino acid sequence having at least 90% sequence identity with SEQ ID NO:21, SEQ ID NO:23, SEQ ID NO:25 or SEQ ID NO:27, for example it can be amino acid sequence having 90%, 91%, 92%, 93%, 94%, 95%, 96%, 97%, or 98% sequence identity with SEQ ID NO:21, SEQ ID NO:23, SEQ ID NO:25 or SEQ ID NO:27.

In one embodiment, the α chain further comprises an α constant region and/or the β chain further comprises a β constant region, preferably, the constant region is a mouse constant region or a human constant region.

For example, the amino acid sequence of the mouse α constant region is represented by SEQ ID NO: 28; and/or
the amino acid sequence of the mouse β constant region is represented by SEQ ID NO: 29; that is, the constant regions of the α chains of the TCRs described above can all have the same constant region. Similarly, the constant regions of the β chains of all the TCRs can also have the same constant region.

The amino acids represented by SEQ ID NO:28 is:

IQNPEPAVYQLKDPRSQDSTLCLFTDFDSQINVPKTMESGTFITDKCVLDM
KAMDSKSNGAIAWSNQTSFTCQDIFKETNATYPSSDVPCDATLTEKSFETDMN
LNFQNLSVMGLRILLLKVAGFNLLMTLRLWSS.

The amino acids represented by SEQ ID NO:29 is:

EDLRNVTPPKVSLFEPSKAEIANKQKATLVCLARGFFPDHVELSWWVNG
KEVHSGVCTDPQAYKESNYSYCLSSRLRVSATFWHNPRNHFRCQVQFHGLSE
EDKWPEGSPKPVTQNISAEAWGRADCGITSASYHQGVLSATILYEILLGKATLY
AVLVSGLVLMAMVKKKNS.

The constant region of the TCR may contain a short linker sequence in which cysteine residues form a disulfide bond, thereby linking the two chains of the TCR. The TCR may have additional cysteine residue(s) in each of the α and β chains such that the TCR comprises two disulfide bonds in the constant regions.

In one embodiment, the amino acid sequence of the variable region of the α chain of the TCR is represented by SEQ ID NO:20, and the amino acid sequence of the variable region of the β chain is represented by SEQ ID NO:21; or the amino acid sequence of the variable region of the α chain is represented by SEQ ID NO:22, and the amino acid sequence of the variable region of the β chain is represented by SEQ ID NO:23; or the amino acid sequence of the variable region of the α chain is represented by SEQ ID NO:24, and the amino acid sequence of the variable region of the β chain is represented by SEQ ID NO:25; or the amino acid sequence of the variable region of the α chain is represented by SEQ ID NO:26, and the amino acid sequence of the variable region of the β chain is represented by SEQ ID NO:27.

In one embodiment, artificial disulfide bonds are introduced between the residues of the constant regions of the α chain and the β chain of the TCR, and the positions of the disulfide bonds that can be introduced are well known to those skilled in the art.

In one embodiment, the TCR is isolated or purified, or is recombinant.

In one embodiment, the TCR is human.

In one embodiment, the TCR is monoclonal.

In one embodiment, the TCR is single chain.

In one embodiment, the TCR comprises two chains.

The TCR can be obtained from a biological source, such as from a cell (such as from a T cell (e.g., a cytotoxic T cell)), a T cell hybridoma, or other publicly available resource, for example, the TCR can be derived from one of a variety of animal species, such as human, mouse, rat or other mammal, such as typically from human.

In some embodiments, the TCR may be in a cell-bound form or in a soluble form, preferably a soluble form.

The TCR in a soluble form refers to a TCR whose hydrophobic core region has undergone mutations, these mutations in the hydrophobic core region are preferably mutations that can improve the stability of the soluble TCR of the present application.

In one embodiment, the amino acid sequence of the α chain variable region of the TCR is represented by SEQ ID NO:20, and the amino acid sequence of the β chain variable region is represented by SEQ ID NO:21; or the amino acid sequence of the α chain variable region is represented by SEQ ID NO:22, and the amino acid sequence of the β chain variable region is represented by SEQ ID NO:23; or the amino acid sequence of the α chain variable region is represented by SEQ ID NO:24, and the amino acid sequence of the β chain variable region is represented by SEQ ID NO:25; or the amino acid sequence of the α chain variable region is represented by SEQ ID NO:26, and the amino acid sequence of the β chain variable region is represented by SEQ ID NO:27.

In one embodiment, the T cell receptor (TCR) described in the present application comprises an α chain comprising a variable region and/or a β chain comprising a variable region, the variable region of the α chain comprises a complementarity determining region 1 (CDR1) with an amino acid sequence of SEQ ID NO:3 or SEQ ID NO:9; and/or a complementarity determining region 2 (CDR2) with an amino acid sequence of SEQ ID NO:4 or SEQ ID NO: 10. In one embodiment, the variable region of the β chain comprises a complementarity determining region 1 (CDR1) with an amino acid sequence of SEQ ID NO:6; and/or a complementarity determining region 2 (CDR2) with an amino acid sequence of SEQ ID NO:7. In one embodiment, the variable region of the α chain comprises a complementary determining region 3 (CDR3) comprising an amino acid sequence of SEQ ID NO:5, SEQ ID NO:11, SEQ ID NO: 13 or SEQ ID NO:15; and/or the variable region of the β chain comprises a complementary determining region 3 (CDR3) comprising an amino acid sequence of SEQ ID NO:8, SEQ ID NO:12, SEQ ID NO:14 or SEQ ID NO:16; preferably, the variable region of the α chain further comprises a first leader sequence; and/or the variable region of the β chain further comprises a second leader sequence; preferably, the amino acid sequence of the variable region of α chain is represented by SEQ ID NO:20, SEQ ID NO:22, SEQ ID NO:24 or SEQ ID NO:26, or is an amino acid sequence having at least 90% sequence identity with SEQ ID NO:20, SEQ ID NO:22, SEQ ID NO:24 or SEQ ID NO:26; and/or the amino acid sequence of the variable region of the β chain is represented by SEQ ID NO:21, SEQ ID NO:23, SEQ ID NO:25 or SEQ ID NO: ID NO:27, or is an amino acid sequence having at least 90% sequence identity with SEQ ID NO:21, SEQ ID NO:23, SEQ ID NO:25 or SEQ ID NO:27; preferably, the α chain further comprises an α constant region and/or the β chain further comprises a β constant region, preferably, the constant region is a mouse constant region or a human constant region. In one embodiment, the TCR is isolated or purified or is recombinant; preferably, the TCR is human; preferably, the TCR is monoclonal; preferably, the TCR is a single chain; preferably, the TCR comprises two chains; preferably, the TCR is in cell-bound form or in soluble form, preferably in soluble form; preferably, the TCR binds to an antigenic short peptide-HLAA1101 complex, and preferably, the amino acid sequence of the antigenic short peptide is represented by SEQ ID NO: 1 or SEQ ID NO:2.

The present application also provides a nucleic acid molecule, which comprises a nucleic acid molecule encoding the TCR or the α chain or β chain of the TCR.

In one embodiment, the nucleotide sequence encoding the α chain comprises the nucleotide sequence of SEQ ID NO: 30, SEQ ID NO: 32, SEQ ID NO: 34 or SEQ ID NO: 36; and/or
the nucleotide sequence encoding the β chain comprises the nucleotide sequence of SEQ ID NO:31, SEQ ID NO:33, SEQ ID NO:35 or SEQ ID NO:37;
Wherein the nucleotide sequence represented by SEQ ID NO:30 is: 
ATGCTCCTGCTGCTCGTCCCAGTGCTCGAGGTGATTTTTACTCTGGGAG
GAACCAGAGCCCAGTCGGTGACCCAGCTTGACAGCCACGTCTCTGTCTCT
GAAGGAACCCCGGTGCTGCTGAGGTGCAACTACTCATCTTCTTATTCACCA
TCTCTCTTCTGGTATGTGCAACACCCCAACAAAGGACTCCAGCTTCTCCTG
AAGTACACATCAGCGGCCACCCTGGTTAAAGGCATCAACGGTTTTGAGGCT
GAATTTAAGAAGAGTGAAACCTCCTTCCACCTGACGAAACCCTCAGCCCAT
ATGAGCGACGCGGCTGAGTACTTCTGTGTTGTGAGTCTTTCTGGTGGCTAC
AATAAGCTGATTTTTGGAGCAGGGACCAGGCTGGCTGTACACCCATATATCC
AGAATCCAGAGCCCGCCGTGTATCAGCTGAAGGACCCAAGGAGCCAGGAT
TCCACCCTGTGCCTGTTCACAGACTTTGATAGCCAGATCAACGTGCCCAAG
ACCATGGAGTCCGGCACCTTCATCACAGACAAGTGCGTGCTGGATATGAAG
GCCATGGACTCTAAGAGCAACGGCGCCATCGCCTGGAGCAATCAGACCTC
CTTCACATGCCAGGATATCTTTAAGGAGACCAATGCCACATATCCTTCCTCT
 
GACGTGCCATGTGATGCCACCCTGACAGAGAAGTCCTTCGAGACCGACAT
GAACCTGAATTTTCAGAACCTGTCTGTGATGGGCCTGCGCATCCTGCTGCT
GAAGGTGGCCGGCTTCAATCTGCTGATGACCCTGAGGCTGTGGAGCTCC.
The nucleotide sequence represented by SEQ ID NO:31 is: 
ATGGGCTTCAGGCTCCTCTGCTGTGTGGCCTTTTGTCTCCTGGGAGCA
GGCCCAGTGGATTCTGGAGTCACACAAACCCCAAAGCACCTGATCACAGC
AACTGGACAGCGAGTGACGCTGAGATGCTCCCCTAGGTCTGGAGACCTCT
CTGTGTACTGGTACCAACAGAGCCTGGACCAGGGCCTCCAGTTCCTCATTC
AGTATTATAATGGAGAAGAGAGAGCAAAAGGAAACATTCTTGAACGATTCT
CCGCACAACAGTTCCCTGACTTGCACTCTGAACTAAACCTGAGCTCTCTGG
AGCTGGGGGACTCAGCTTTGTATTTCTGTGCCAGCAGCGTGACAGGGAGT
GGCTACACCTTCGGTTCGGGGACCAGGTTAACCGTTGTAGAGGATCTGAGG
AACGTGACACCCCCTAAGGTGTCTCTGTTCGAGCCCAGCAAGGCCGAGAT
CGCCAATAAGCAGAAGGCCACCCTGGTGTGCCTGGCAAGGGGCTTCTTTC
CTGATCACGTGGAGCTGTCTTGGTGGGTGAACGGCAAGGAGGTGCACAGC
GGCGTGTGCACCGACCCACAGGCCTACAAGGAGTCCAATTACTCTTATTGT
CTGAGCTCCCGGCTGAGAGTGTCCGCCACATTTTGGCACAACCCTAGAAAT
CACTTCAGGTGCCAGGTGCAGTTTCACGGCCTGAGCGAGGAGGATAAGTG
GCCAGAGGGATCCCCAAAGCCTGTGACCCAGAACATCTCTGCCGAGGCAT
GGGGAAGGGCAGACTGTGGAATCACATCCGCCTCTTATCACCAGGGCGTG
CTGAGCGCCACCATCCTGTACGAGATCCTGCTGGGCAAGGCCACACTGTAT
GCCGTGCTGGTGAGCGGCCTGGTGCTGATGGCCATGGTGAAGAAGAAGAA
CTCC.
The nucleotide sequence represented by SEQ ID NO:32 is: 
ATGCTACTCATCACATCAATGTTGGTCTTATGGATGCAATTGTCACAGG
TGAATGGACAACAGGTAATGCAAATTCCTCAGTACCAGCATGTACAAGAAG
GAGAGGACTTCACCACGTACTGCAATTCCTCAACTACTTTAAGCAATATACA
GTGGTATAAGCAAAGGCCTGGTGGACATCCCGTTTTTTTGATACAGTTAGTG
AAGAGTGGAGAAGTGAAGAAGCAGAAAAGACTGACATTTCAGTTTGGAG
 
AAGCAAAAAAGAACAGCTCCCTGCACATCACAGCCACCCAGACTACAGAT
GTAGGAACCTACTTCTGTGCAGGGCCGAAAATCACGGGAGGAGGAAACAA
ACTCACCTTTGGGACAGGCACTCAGCTAAAAGTGGAACTCAATATCCAGAA
TCCAGAGCCCGCCGTGTATCAGCTGAAGGACCCAAGGAGCCAGGATTCCA
CCCTGTGCCTGTTCACAGACTTTGATAGCCAGATCAACGTGCCCAAGACCA
TGGAGTCCGGCACCTTCATCACAGACAAGTGCGTGCTGGATATGAAGGCCA
TGGACTCTAAGAGCAACGGCGCCATCGCCTGGAGCAATCAGACCTCCTTCA
CATGCCAGGATATCTTTAAGGAGACCAATGCCACATATCCTTCCTCTGACGT
GCCATGTGATGCCACCCTGACAGAGAAGTCCTTCGAGACCGACATGAACCT
GAATTTTCAGAACCTGTCTGTGATGGGCCTGCGCATCCTGCTGCTGAAGGT
GGCCGGCTTCAATCTGCTGATGACCCTGAGGCTGTGGAGCTCC.
The nucleotide sequence represented by SEQ ID NO:33 is: 
ATGGGCTTCAGGCTCCTCTGCTGTGTGGCCTTTTGTCTCCTGGGAGCA
GGCCCAGTGGATTCTGGAGTCACACAAACCCCAAAGCACCTGATCACAGC
AACTGGACAGCGAGTGACGCTGAGATGCTCCCCTAGGTCTGGAGACCTCT
CTGTGTACTGGTACCAACAGAGCCTGGACCAGGGCCTCCAGTTCCTCATTC
AGTATTATAATGGAGAAGAGAGAGCAAAAGGAAACATTCTTGAACGATTCT
CCGCACAACAGTTCCCTGACTTGCACTCTGAACTAAACCTGAGCTCTCTGG
AGCTGGGGGACTCAGCTTTGTATTTCTGTGCCAGCAGCGTAGGCGGGGGCC
CTAACTATGGCTACACCTTCGGTTCGGGGACCAGGTTAACCGTTGTAGAGG
ATCTGAGGAACGTGACACCCCCTAAGGTGTCTCTGTTCGAGCCCAGCAAG
GCCGAGATCGCCAATAAGCAGAAGGCCACCCTGGTGTGCCTGGCAAGGGG
CTTCTTTCCTGATCACGTGGAGCTGTCTTGGTGGGTGAACGGCAAGGAGGT
GCACAGCGGCGTGTGCACCGACCCACAGGCCTACAAGGAGTCCAATTACT
CTTATTGTCTGAGCTCCCGGCTGAGAGTGTCCGCCACATTTTGGCACAACC
CTAGAAATCACTTCAGGTGCCAGGTGCAGTTTCACGGCCTGAGCGAGGAG
GATAAGTGGCCAGAGGGATCCCCAAAGCCTGTGACCCAGAACATCTCTGC
CGAGGCATGGGGAAGGGCAGACTGTGGAATCACATCCGCCTCTTATCACCA
GGGCGTGCTGAGCGCCACCATCCTGTACGAGATCCTGCTGGGCAAGGCCA
 
CACTGTATGCCGTGCTGGTGAGCGGCCTGGTGCTGATGGCCATGGTGAAGA
AGAAGAACTCC.
The nucleotide sequence represented by SEQ ID NO:34 is: 
ATGCTACTCATCACATCAATGTTGGTCTTATGGATGCAATTGTCACAGG
TGAATGGACAACAGGTAATGCAAATTCCTCAGTACCAGCATGTACAAGAAG
GAGAGGACTTCACCACGTACTGCAATTCCTCAACTACTTTAAGCAATATACA
GTGGTATAAGCAAAGGCCTGGTGGACATCCCGTTTTTTTGATACAGTTAGTG
AAGAGTGGAGAAGTGAAGAAGCAGAAAAGACTGACATTTCAGTTTGGAG
AAGCAAAAAAGAACAGCTCCCTGCACATCACAGCCACCCAGACTACAGAT
GTAGGAACCTACTTCTGTGCAGGCCCCATACTCACGGGAGGAGGAAACAA
ACTCACCTTTGGGACAGGCACTCAGCTAAAAGTGGAACTCAATATCCAGAA
TCCAGAGCCCGCCGTGTATCAGCTGAAGGACCCAAGGAGCCAGGATTCCA
CCCTGTGCCTGTTCACAGACTTTGATAGCCAGATCAACGTGCCCAAGACCA
TGGAGTCCGGCACCTTCATCACAGACAAGTGCGTGCTGGATATGAAGGCCA
TGGACTCTAAGAGCAACGGCGCCATCGCCTGGAGCAATCAGACCTCCTTCA
CATGCCAGGATATCTTTAAGGAGACCAATGCCACATATCCTTCCTCTGACGT
GCCATGTGATGCCACCCTGACAGAGAAGTCCTTCGAGACCGACATGAACCT
GAATTTTCAGAACCTGTCTGTGATGGGCCTGCGCATCCTGCTGCTGAAGGT
GGCCGGCTTCAATCTGCTGATGACCCTGAGGCTGTGGAGCTCC.
The nucleotide sequence represented by SEQ ID NO:35 is: 
ATGGGCTTCAGGCTCCTCTGCTGTGTGGCCTTTTGTCTCCTGGGAGCA
GGCCCAGTGGATTCTGGAGTCACACAAACCCCAAAGCACCTGATCACAGC
AACTGGACAGCGAGTGACGCTGAGATGCTCCCCTAGGTCTGGAGACCTCT
CTGTGTACTGGTACCAACAGAGCCTGGACCAGGGCCTCCAGTTCCTCATTC
AGTATTATAATGGAGAAGAGAGAGCAAAAGGAAACATTCTTGAACGATTCT
CCGCACAACAGTTCCCTGACTTGCACTCTGAACTAAACCTGAGCTCTCTGG
AGCTGGGGGACTCAGCTTTGTATTTCTGTGCCAGCGGGCTTTCAGGACCGA
ACACCGGGGAGCTGTTTTTTGGAGAAGGCTCTAGGCTGACCGTACTGGAG
GATCTGAGGAACGTGACACCCCCTAAGGTGTCTCTGTTCGAGCCCAGCAA
 
GGCCGAGATCGCCAATAAGCAGAAGGCCACCCTGGTGTGCCTGGCAAGGG
GCTTCTTTCCTGATCACGTGGAGCTGTCTTGGTGGGTGAACGGCAAGGAGG
TGCACAGCGGCGTGTGCACCGACCCACAGGCCTACAAGGAGTCCAATTAC
TCTTATTGTCTGAGCTCCCGGCTGAGAGTGTCCGCCACATTTTGGCACAAC
CCTAGAAATCACTTCAGGTGCCAGGTGCAGTTTCACGGCCTGAGCGAGGA
GGATAAGTGGCCAGAGGGATCCCCAAAGCCTGTGACCCAGAACATCTCTG
CCGAGGCATGGGGAAGGGCAGACTGTGGAATCACATCCGCCTCTTATCACC
AGGGCGTGCTGAGCGCCACCATCCTGTACGAGATCCTGCTGGGCAAGGCC
ACACTGTATGCCGTGCTGGTGAGCGGCCTGGTGCTGATGGCCATGGTGAAG
AAGAAGAACTCC.
The nucleotide sequence represented by SEQ ID NO:36 is: 
ATGCTACTCATCACATCAATGTTGGTCTTATGGATGCAATTGTCACAGG
TGAATGGACAACAGGTAATGCAAATTCCTCAGTACCAGCATGTACAAGAAG
GAGAGGACTTCACCACGTACTGCAATTCCTCAACTACTTTAAGCAATATACA
GTGGTATAAGCAAAGGCCTGGTGGACATCCCGTTTTTTTGATACAGTTAGTG
AAGAGTGGAGAAGTGAAGAAGCAGAAAAGACTGACATTTCAGTTTGGAG
AAGCAAAAAAGAACAGCTCCCTGCACATCACAGCCACCCAGACTACAGAT
GTAGGAACCTACTTCTGTGCAGGGCCGGTACTCACGGGAGGAGGAAACAA
ACTCACCTTTGGGACAGGCACTCAGCTAAAAGTGGAACTCAATATCCAGAA
TCCAGAGCCCGCCGTGTATCAGCTGAAGGACCCAAGGAGCCAGGATTCCA
CCCTGTGCCTGTTCACAGACTTTGATAGCCAGATCAACGTGCCCAAGACCA
TGGAGTCCGGCACCTTCATCACAGACAAGTGCGTGCTGGATATGAAGGCCA
TGGACTCTAAGAGCAACGGCGCCATCGCCTGGAGCAATCAGACCTCCTTCA
CATGCCAGGATATCTTTAAGGAGACCAATGCCACATATCCTTCCTCTGACGT
GCCATGTGATGCCACCCTGACAGAGAAGTCCTTCGAGACCGACATGAACCT
GAATTTTCAGAACCTGTCTGTGATGGGCCTGCGCATCCTGCTGCTGAAGGT
GGCCGGCTTCAATCTGCTGATGACCCTGAGGCTGTGGAGCTCC.
The nucleotide sequence represented by SEQ ID NO:37 is:

ATGGGCTTCAGGCTCCTCTGCTGTGTGGCCTTTTGTCTCCTGGGAGCA

GGCCCAGTGGATTCTGGAGTCACACAAACCCCAAAGCACCTGATCACAGC
AACTGGACAGCGAGTGACGCTGAGATGCTCCCCTAGGTCTGGAGACCTCT
CTGTGTACTGGTACCAACAGAGCCTGGACCAGGGCCTCCAGTTCCTCATTC
AGTATTATAATGGAGAAGAGAGAGCAAAAGGAAACATTCTTGAACGATTCT
CCGCACAACAGTTCCCTGACTTGCACTCTGAACTAAACCTGAGCTCTCTGG
AGCTGGGGGACTCAGCTTTGTATTTCTGTGCCAGCAGCGTGGGGGGACCG
AACACCGGGGAGCTGTTTTTTGGAGAAGGCTCTAGGCTGACCGTACTGGA
GGATCTGAGGAACGTGACACCCCCTAAGGTGTCTCTGTTCGAGCCCAGCA
AGGCCGAGATCGCCAATAAGCAGAAGGCCACCCTGGTGTGCCTGGCAAGG
GGCTTCTTTCCTGATCACGTGGAGCTGTCTTGGTGGGTGAACGGCAAGGAG
GTGCACAGCGGCGTGTGCACCGACCCACAGGCCTACAAGGAGTCCAATTA
CTCTTATTGTCTGAGCTCCCGGCTGAGAGTGTCCGCCACATTTTGGCACAA
CCCTAGAAATCACTTCAGGTGCCAGGTGCAGTTTCACGGCCTGAGCGAGG
AGGATAAGTGGCCAGAGGGATCCCCAAAGCCTGTGACCCAGAACATCTCT
GCCGAGGCATGGGGAAGGGCAGACTGTGGAATCACATCCGCCTCTTATCAC
CAGGGCGTGCTGAGCGCCACCATCCTGTACGAGATCCTGCTGGGCAAGGC
CACACTGTATGCCGTGCTGGTGAGCGGCCTGGTGCTGATGGCCATGGTGAA
GAAGAAGAACTCC.

In one embodiment, the nucleotide sequence encoding the α chain and/or the nucleotide sequence encoding the β chain is codon optimized. Typically, codon optimization involves balancing the percentage of selected codons with the abundance of disclosed human transfer RNAs, such that none of them are overloaded or restricted. In some cases, this may be necessary because most amino acids are encoded by more than one codon and codon usage varies among organisms. Differences in codon usage between transfected genes and host cells may affect protein expression and immunogenicity of the nucleic acid constructs. Typically, for codon optimization, codons are chosen to select for those codons that are balanced in human usage frequency. Generally, the redundancy of amino acid codons allows different codons to encode one amino acid. In some embodiments, when selecting codons for replacement, it may be necessary for the resulting mutation to be silent, so that the codon change does not affect the amino acid sequence. Often, the last nucleotide of a codon can remain unchanged without affecting the amino acid sequence.

The present application provides a vector, which comprises the nucleic acid molecule described above.

For example, one or more nucleic acids encoding one or both chains of the above-mentioned TCR are cloned into one or more suitable expression vectors. The expression vector can be any suitable recombinant expression vector and can be used to transform or transfect any suitable host. Suitable vectors include those designed for propagation and amplification or for expression or for both, such as plasmids and viruses.

The vector may contain regulatory sequences (such as transcriptional and translational initiation and termination codons) that are specific for the type of host (e.g., bacteria, fungi, plants, or animals) into which the vector is to be introduced, as appropriate and taking into consideration whether the vector is DNA-based or RNA-based. The vector may also contain a non-native promoter operably linked to the nucleotide sequence encoding the TCR. The promoter can be a non-viral promoter or a viral promoter, such as the cytomegalovirus (CMV) promoter, the SV40 promoter, the RSV promoter, and the promoter found in the long terminal repeat of the murine stem cell virus, and other promoters known to the skilled in the art are also contemplated.

The vector is an expression vector, preferably a viral vector, preferably a retroviral vector, and more preferably a lentivirus vector.

The present application also provides a host cell comprising the above-mentioned nucleic acid molecule. In order to recombinantly produce TCR, the nucleic acid encoding TCR can be isolated and inserted into one or more vectors for further cloning and/or expression in the host cell. Such nucleic acid can be readily isolated and sequenced using conventional techniques (e.g., by using oligonucleotide probes that are capable of binding specifically to genes encoding the α and β chains of the TCR). In some embodiments, a method for preparing a TCR is provided, wherein the method comprises culturing a host cell comprising a nucleic acid encoding a TCR as provided above under conditions suitable for expressing the TCR molecule, and optionally recovering the TCR from the host cell (or host cell culture medium).

The host cell refers to a cell into which exogenous nucleic acid has been introduced, including the progeny of such a cell. Host cells include transformants and transformed cells, including the primary transformed cell and progeny derived therefrom, regardless of the number of passages. The progeny may not be completely identical to the parent cell in terms of nucleic acid content, but may contain mutations.

The present application also provides an engineered cell, which comprises the aforementioned T cell receptor (TCR), nucleic acid molecule, or the vector.

In one embodiment, the TCR is heterologous to the cell.

In one embodiment, the engineered cell is a cell line.

In one embodiment, the engineered cell is a primary cell obtained from a subject, preferably the subject is a mammalian subject, preferably a human.

In one embodiment, the engineered cell is a T cell, preferably a T cell isolated from peripheral blood.

In one embodiment, the T cell is CD8⁺ or CD4⁺.

The engineered cell may be, for example, a cell population or a genetically engineered cell expressing a TCR, the cell is typically a eukaryotic cell, such as a mammalian cell, and typically a human cell. In some embodiments, the cell is derived from blood, bone marrow, lymph or a lymphoid organ and is a cell of the immune system, such as a cell of innate immunity or adaptive immunity, for example a myeloid or lymphoid cell (including lymphocytes, typically a T cell and/or an NK cell). Other exemplary cells include stem cells, such as multipotent stem cells and pluripotent stem cells, including induced pluripotent stem cells (iPSCs). The cells are typically primary cells, such as those isolated directly from a subject and/or isolated from a subject and frozen. In some embodiments, the cells include one or more subsets of T cells or other cell types, such as the entire T cell population, CD+ cells, CD8+ cells, and subsets thereof.

Subtypes and subpopulations of T cells and/or CD+ and/or CD8+ T cells include naive T (T_{N}) cells, effector T cells (T_{EFF}), memory T cells and their subtypes (such as stem cell memory T cells (T_{SCM}), central memory T cells (T_{CM}), effector memory T cells (T_{EM}), or terminally differentiated effector memory T cells, tumor infiltrating lymphocytes (TIL), immature T cells, mature T cells, helper T cells, cytotoxic T cells, mucosal-associated invariant T (MAIT) cells, naturally occurring and adaptive regulatory T (Treg) cells, etc.

In some embodiments, the engineered cells are natural killer (NK) cells, preferably, the cells are monocytes or granulocytes, such as myeloid cells, macrophages, neutrophils, dendritic cells, mast cells, eosinophils and/or basophils.

The present application provides a method for producing the engineered cells described above, comprising introducing the aforementioned nucleic acid molecules or vectors into cells *in vitro* or *ex vivo.*

The vector is a viral vector, and the introduction is performed by transduction.

The present application provides a pharmaceutical composition, comprising the aforementioned T cell receptor (TCR), nucleic acid molecule, vector, or the engineered cell. In one embodiment, it further comprises a pharmaceutically acceptable carrier or adjuvant.

The pharmaceutically acceptable carrier or adjuvant refers to ingredients in a pharmaceutical composition other than the active ingredient that are non-toxic to the subject. Pharmaceutically acceptable carriers or adjuvants include, but are not limited to, buffers, excipients, stabilizers or preservatives.

The pharmaceutical composition may utilize time-release, delayed-release, and sustained-release delivery systems, such that the delivery of the composition occurs prior to sensitization of the site to be treated and allows sufficient time to induce sensitization. Many types of release delivery systems are available and known. Such systems can avoid repeated administration of the composition, thereby increasing convenience for subjects and physicians.

The present application provides the use of the aforementioned T cell receptor (TCR), nucleic acid molecule, vector, engineered cell or the pharmaceutical composition in the preparation of a medicament for treating HPV-related diseases.

In one embodiment, the HPV-related disease is chronic HPV infection, cervical intraepithelial neoplasia, cervical cancer, head and neck cancer, anal cancer, penile cancer, vaginal cancer or vulvar cancer, etc.

The present application provides a method for treating HPV-related diseases, comprising administering the aforementioned T cell receptor (TCR), nucleic acid molecule, vector, engineered cell, or the aforementioned pharmaceutical composition to a subject in need thereof. In one embodiment, the HPV-related disease is chronic HPV infection, cervical intraepithelial neoplasia, cervical cancer, head and neck cancer, anal cancer, penile cancer, vaginal cancer or vulvar cancer.

The TCR described in the present application can specifically bind to the antigenic short peptide of HPV-positive tumor cells. The T cells transduced with the TCR can be specifically activated and have a strong killing effect on target cells. The TCR can be used for immunotherapy of HPV-positive tumors such as cervical cancer.

The specific T cell receptors (TCRs) can be screen out by using antigenic short peptides in the present application, and the T cells transduced with the TCRs can be specifically activated and have a strong killing effect on tumor cells expressing A1101 and HPV, and they can be used for immunotherapy of HPV-positive tumors such as cervical cancer.

The T cells transduced with the TCR in the present application have a strong activation response to cell lines expressing E6, but have no activation response to cell lines not expressing E6, and have a strong killing effect to cell lines expressing E6, and can effectively inhibit the growth of E6-positive tumors.

The T cells transduced with the TCR in the present application exhibit significant cytotoxic activity against primary cervical cancer organoid cells, and can effectively inhibit the growth of E6-positive tumors.

### EXAMPLE

The present application provides a general and/or specific description of the materials and experimental methods used in the experiments. In the following Examples, unless otherwise specified, % means wt%, i.e., weight percentage. If the manufacturer of the reagents or instruments used is not indicated, they are all conventional reagent products that can be obtained through commercial purchase.

### Example 1. Cloning of T cells specific to TTLEQQYNK and GTTLEQQYNK antigenic short peptides

The synthetic short peptides TTLEQQYNK (SEQ ID NO: 1, Jiangsu GenScript Biotech Co., Ltd.) and GTTLEQQYNK (SEQ ID NO: 2, Jiangsu GenScript Biotech Co., Ltd.) were used to stimulate peripheral blood lymphocytes from healthy volunteers with the genotype of HLA-A*11:01. The TTLEQQYNK short peptide and the GTTLEQQYNK short peptide were respectively renatured with biotinylated HLA-A*11:01 to prepare pHLA monomers. These monomers were combined with PE-labeled streptavidin (Becton, Dickinson and Company, Cat. No. 554061) to form PE-labeled tetramers, and the tetramer and CD8 double-positive cells were detected by flow cytometry; wherein the preparation method of pHLA monomer and tetramer refers to the protocol published by the NIH Tetramer Core Facility, and the specific steps can be found on the website https://tetramer.yerkes.emory.edu/support/protocols#1. The specific procedures are as follows:
**(1) Reagents**
   Experimental culture medium: 10% FBS (ThermoFisher, Cat. No. 10099-044), RPMI1640 (ThermoFisher, Cat. No. C11875500BT), 10% HS (Gemni, Cat. No. 100512), TexMACS (Mitenyi, Cat. No. 170-076-309)
**(2) Methods**
   PBMCs were separated from the peripheral blood of healthy volunteers by density gradient centrifugation, and CD14 positive cells were separated from PBMCs for inducing dendritic cells (DCs). CD14-positive cells were cultured in the culture medium (1640+10% AuFBS+1% PS+800IU/ml GM-CSF+500IU/ml IL-4) until the third day, and then 1ml of culture medium (comprising 1600IU/ml GM-CSF+1000IU/ml IL-4) was supplemented. On the fifth day, DC maturation-inducing factors (10ng/ml IL-1β, 10ng/ml IL-6, 10ng/ml TNF-α, and 1ug/ml PGE2) were added, and the cells were cultured for another two days to obtain mature DCs. Mature DCs were used to load TTLEQQYNK short peptide and GTTLEQQYNK at a final concentration of 20 µg/ml, and were then used to stimulate naive CD8 positive T cells isolated from PBMCs (culture medium: TexMACS + 10% HS + 1% PS + 60ng/ml IL21 + 10IU/ml IL2/7/15). After three rounds of stimulation, tetramer and CD8 double-positive T cells (i.e., the antigen-specific T cells) were sorted out using flow cytometry. The TCR α chain and β chain were amplified respectively using the One-Step RT-PCR kit (QIAGEN, Cat. No. 210212) from the sorted CD8 and tetramer double-positive single cells, and the PCR products were sent for sequencing. By comparing the sequencing results with the sequences in the public database of IMGT (International Immunogenetics Information System), the nucleotide sequences of the TCR α chain variable region and the β chain variable region and the information of their CDR1, CDR2, and CDR3 can be obtained.
**(3) Results**

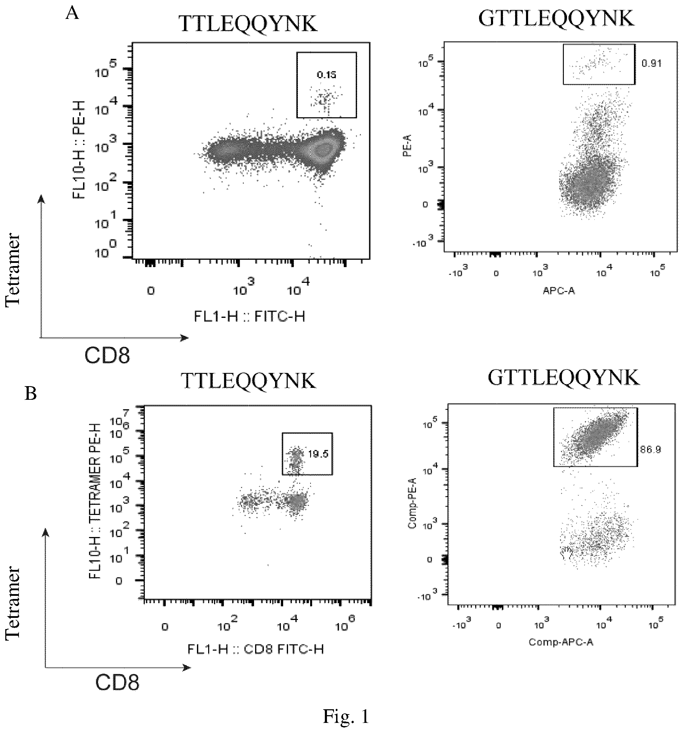

T cells specific to TTLEQQYNK and GTTLEQQYNK antigenic short peptides were cloned using the method described above, and the cloned antigenic short peptide-specific T cells were analyzed by flow cytometry. The results of T cell flow cytometry after two and three rounds of stimulation with TTLEQQYNK and GTTLEQQYNK antigenic short peptides are shown in Fig. 1, wherein Fig. 1A is a schematic diagram of the flow cytometry results for T cells after two rounds of stimulation, and Fig. 1B is a schematic diagram of the flow cytometry results for T cell after three rounds of stimulation.

As can be seen from Fig. 1, T cells specific to TTLEQQYNK and GTTLEQQYNK antigenic short peptides had been successfully cloned, wherein part of the TCR sequence obtained by TTLEQQYNK is described in detail in Example 2 below.

### Example 2: Construction of TTLEQQYNK antigenic short peptide-specific TCR lentiviral vector and lentiviral packaging

### (1) Construction of TCR lentiviral vector

The CD8 and tetramer double-positive cells in Example 1 were sorted by flow cytometry to obtain single cells; the α chain and β chain of the TCR were amplified respectively using the One-Step RT-PCR kit (QIAGEN, Cat. No. 210212) from the sorted single cells, and the PCR products were sequenced. By comparing the sequencing results with the sequences in the public database of IMGT (International Immunogenetics Information System), the nucleotide sequences of the α chain variable region and the β chain variable region of the TCR and the information of their CDR1, CDR2, and CDR3 can be obtained. The nucleotide sequences of the α chain variable region and β chain variable region of four TCRs (TCR010, TCR013, TCR028, and TCR090) and the information of the CDR1, CDR2, and CDR3 thereof were obtained, which are shown below:
the nucleotide sequence of the α chain variable region of TCR010 is shown as SEQ ID NO: 39, and the nucleotide sequence is as follows: 
ATGCTCCTGCTGCTCGTCCCAGTGCTCGAGGTGATTTTTACTCTGGGAG
GAACCAGAGCCCAGTCGGTGACCCAGCTTGACAGCCACGTCTCTGTCTCT
GAAGGAACCCCGGTGCTGCTGAGGTGCAACTACTCATCTTCTTATTCACCA
TCTCTCTTCTGGTATGTGCAACACCCCAACAAAGGACTCCAGCTTCTCCTG
AAGTACACATCAGCGGCCACCCTGGTTAAAGGCATCAACGGTTTTGAGGCT
GAATTTAAGAAGAGTGAAACCTCCTTCCACCTGACGAAACCCTCAGCCCAT
ATGAGCGACGCGGCTGAGTACTTCTGTGTTGTGAGTCTTTCTGGTGGCTAC
AATAAGCTGATTTTTGGAGCAGGGACCAGGCTGGCTGTACACCCATAT;
The nucleotide sequence of the β chain variable region is represented by SEQ ID NO: 40, and the nucleotide sequence is as follows: 
ATGGGCTTCAGGCTCCTCTGCTGTGTGGCCTTTTGTCTCCTGGGAGCA
GGCCCAGTGGATTCTGGAGTCACACAAACCCCAAAGCACCTGATCACAGC
AACTGGACAGCGAGTGACGCTGAGATGCTCCCCTAGGTCTGGAGACCTCT
CTGTGTACTGGTACCAACAGAGCCTGGACCAGGGCCTCCAGTTCCTCATTC
AGTATTATAATGGAGAAGAGAGAGCAAAAGGAAACATTCTTGAACGATTCT
CCGCACAACAGTTCCCTGACTTGCACTCTGAACTAAACCTGAGCTCTCTGG
AGCTGGGGGACTCAGCTTTGTATTTCTGTGCCAGCAGCGTGACAGGGAGT
GGCTACACCTTCGGTTCGGGGACCAGGTTAACCGTTGTA;
The amino acid sequence of the complementarity determining region 1 (CDR1) of the **α chain** is represented by SEQ ID NO: 3: SSYSPS;
   The amino acid sequence of the complementarity determining region 2 (CDR2) is represented by SEQ ID NO: 4: YTSAATLV;
   The amino acid sequence of the complementarity determining region 3 (CDR3) is represented by SEQ ID NO: 5: VVSLSGGYNKLI.
The amino acid sequence of the complementarity determining region 1 (CDR1) of the **β chain** is represented by SEQ ID NO: 6: SGDLS;
   The amino acid sequence of the complementarity determining region 2 (CDR2) is represented by SEQ ID NO: 7: YYNGEE;
   The amino acid sequence of the complementarity determining region 3 (CDR3) is represented by SEQ ID NO: 8: ASSVTGSGYT.
The nucleotide sequence of the **α chain** variable region of TCR013 is represented by SEQ ID NO: 41: 
ATGCTACTCATCACATCAATGTTGGTCTTATGGATGCAATTGTCACAGG
TGAATGGACAACAGGTAATGCAAATTCCTCAGTACCAGCATGTACAAGAAG
GAGAGGACTTCACCACGTACTGCAATTCCTCAACTACTTTAAGCAATATACA
GTGGTATAAGCAAAGGCCTGGTGGACATCCCGTTTTTTTGATACAGTTAGTG
AAGAGTGGAGAAGTGAAGAAGCAGAAAAGACTGACATTTCAGTTTGGAG
AAGCAAAAAAGAACAGCTCCCTGCACATCACAGCCACCCAGACTACAGAT
 
GTAGGAACCTACTTCTGTGCAGGGCCGAAAATCACGGGAGGAGGAAACAA
ACTCACCTTTGGGACAGGCACTCAGCTAAAAGTGGAACTCAAT;
The nucleotide sequence of the **β chain** variable region is represented by SEQ ID NO: 42: 
ATGGGCTTCAGGCTCCTCTGCTGTGTGGCCTTTTGTCTCCTGGGAGCA
GGCCCAGTGGATTCTGGAGTCACACAAACCCCAAAGCACCTGATCACAGC
AACTGGACAGCGAGTGACGCTGAGATGCTCCCCTAGGTCTGGAGACCTCT
CTGTGTACTGGTACCAACAGAGCCTGGACCAGGGCCTCCAGTTCCTCATTC
AGTATTATAATGGAGAAGAGAGAGCAAAAGGAAACATTCTTGAACGATTCT
CCGCACAACAGTTCCCTGACTTGCACTCTGAACTAAACCTGAGCTCTCTGG
AGCTGGGGGACTCAGCTTTGTATTTCTGTGCCAGCAGCGTAGGCGGGGGCC
CTAACTATGGCTACACCTTCGGTTCGGGGACCAGGTTAACCGTTGTA;
The amino acid sequence of the complementarity determining region 1 (CDR1) of the **α chain** is represented by SEQ ID NO:9: TTLSN;
   The amino acid sequence of the complementarity determining region 2 (CDR2) is represented by SEQ ID NO: 10: LVKSGEV;
   The amino acid sequence of the complementarity determining region 3 (CDR3) is represented by SEQ ID NO: 11: AGPKITGGGNKLT;
The amino acid sequence of the complementarity determining region 1 (CDR1) of the **β chain** is represented by SEQ ID NO: 6: SGDLS;
   The amino acid sequence of the complementarity determining region 2 (CDR2) is represented by SEQ ID NO:7: YYNGEE;
   The amino acid sequence of the complementarity determining region 3 (CDR3) is represented by SEQ ID NO: 12: ASSVGGGPNYGYT.
The nucleotide sequence of the **α chain** variable region sequence of TCR028 is represented by SEQ ID NO: 43: 
ATGCTACTCATCACATCAATGTTGGTCTTATGGATGCAATTGTCACAGG
TGAATGGACAACAGGTAATGCAAATTCCTCAGTACCAGCATGTACAAGAAG
GAGAGGACTTCACCACGTACTGCAATTCCTCAACTACTTTAAGCAATATACA
 
GTGGTATAAGCAAAGGCCTGGTGGACATCCCGTTTTTTTGATACAGTTAGTG
AAGAGTGGAGAAGTGAAGAAGCAGAAAAGACTGACATTTCAGTTTGGAG
AAGCAAAAAAGAACAGCTCCCTGCACATCACAGCCACCCAGACTACAGAT
GTAGGAACCTACTTCTGTGCAGGCCCCATACTCACGGGAGGAGGAAACAA
ACTCACCTTTGGGACAGGCACTCAGCTAAAAGTGGAACTCAAT;
The nucleotide sequence of the **β chain** variable region is represented by SEQ ID NO: 44: 
ATGGGCTTCAGGCTCCTCTGCTGTGTGGCCTTTTGTCTCCTGGGAGCA
GGCCCAGTGGATTCTGGAGTCACACAAACCCCAAAGCACCTGATCACAGC
AACTGGACAGCGAGTGACGCTGAGATGCTCCCCTAGGTCTGGAGACCTCT
CTGTGTACTGGTACCAACAGAGCCTGGACCAGGGCCTCCAGTTCCTCATTC
AGTATTATAATGGAGAAGAGAGAGCAAAAGGAAACATTCTTGAACGATTCT
CCGCACAACAGTTCCCTGACTTGCACTCTGAACTAAACCTGAGCTCTCTGG
AGCTGGGGGACTCAGCTTTGTATTTCTGTGCCAGCGGGCTTTCAGGACCGA
ACACCGGGGAGCTGTTTTTTGGAGAAGGCTCTAGGCTGACCGTACTG;
The amino acid sequence of the complementarity determining region 1 (CDR1) of the **α chain** is represented by SEQ ID NO: 9: TTLSN;
   The amino acid sequence of the complementarity determining region 2 (CDR2) is represented by SEQ ID NO: 10: LVKSGEV;
   The amino acid sequence of the complementarity determining region 3 (CDR3) is represented by SEQ ID NO: 13: AGPILTGGGNKLT;
The amino acid sequence of the complementarity determining region 1 (CDR1) of the **β chain** is represented by SEQ ID NO: 6: SGDLS;
   The amino acid sequence of the complementarity determining region 2 (CDR2) is represented by SEQ ID NO: 7: YYNGEE;
   The amino acid sequence of the complementarity determining region 3 (CDR3) is represented by SEQ ID NO: 14: ASGLSGPNTGELF.
The nucleotide sequence of the **α chain** variable region of TCR090 is represented by SEQ ID NO: 45: 
ATGCTACTCATCACATCAATGTTGGTCTTATGGATGCAATTGTCACAGG
TGAATGGACAACAGGTAATGCAAATTCCTCAGTACCAGCATGTACAAGAAG
GAGAGGACTTCACCACGTACTGCAATTCCTCAACTACTTTAAGCAATATACA
GTGGTATAAGCAAAGGCCTGGTGGACATCCCGTTTTTTTGATACAGTTAGTG
AAGAGTGGAGAAGTGAAGAAGCAGAAAAGACTGACATTTCAGTTTGGAG
AAGCAAAAAAGAACAGCTCCCTGCACATCACAGCCACCCAGACTACAGAT
GTAGGAACCTACTTCTGTGCAGGGCCGGTACTCACGGGAGGAGGAAACAA
ACTCACCTTTGGGACAGGCACTCAGCTAAAAGTGGAACTCAAT;
The nucleotide sequence of the **β chain** variable region is represented by SEQ ID NO: 46: 
ATGGGCTTCAGGCTCCTCTGCTGTGTGGCCTTTTGTCTCCTGGGAGCA
GGCCCAGTGGATTCTGGAGTCACACAAACCCCAAAGCACCTGATCACAGC
AACTGGACAGCGAGTGACGCTGAGATGCTCCCCTAGGTCTGGAGACCTCT
CTGTGTACTGGTACCAACAGAGCCTGGACCAGGGCCTCCAGTTCCTCATTC
AGTATTATAATGGAGAAGAGAGAGCAAAAGGAAACATTCTTGAACGATTCT
CCGCACAACAGTTCCCTGACTTGCACTCTGAACTAAACCTGAGCTCTCTGG
AGCTGGGGGACTCAGCTTTGTATTTCTGTGCCAGCAGCGTGGGGGGACCG
AACACCGGGGAGCTGTTTTTTGGAGAAGGCTCTAGGCTGACCGTACTG;
The amino acid sequence of the complementarity determining region 1 (CDR1) of the **α chain** is represented by SEQ ID NO: 9: TTLSN;
   The amino acid sequence of the complementarity determining region 2 (CDR2) is represented by SEQ ID NO: 10: LVKSGEV;
   The amino acid sequence of the complementarity determining region 3 (CDR3) is represented by SEQ ID NO: 15: AGPVLTGGGNKLT;
The amino acid sequence of the complementarity determining region 1 (CDR1) of the **β chain** is represented by SEQ ID NO: 6: SGDLS;
   The amino acid sequence of the complementarity determining region 2 (CDR2) is represented by SEQ ID NO:7: YYNGEE;
   The amino acid sequence of the complementarity determining region 3 (CDR3) is represented by SEQ ID NO: 16: ASSVGGPNTGELF.
The α and β chain variable region sequences of the TTLEQQYNK TCR were cloned into a pLKO-based expression plasmid (Addgene), and the α or β variable reagion was cloned into a pLKO-based expression plasmid comprising the mouse α or β constant region using the standard method of the multi-fragment recombination cloning kit (Vazyme Biotech Co., Ltd, Cat. No. C113), and the connected plasmids were transformed into competent E. coli strain Stbl3 cells (Shanghai Weidi Biotech Co., Ltd.) and inoculated on LB/agar plates containing 100 µg/ml ampicillin. After overnight incubation at 37°C, a single colony was picked and grown overnight by shaking at 37°C in 10 ml of LB containing 100 µg/ml ampicillin. The cloned plasmids were purified using a miniprep medium sized kit (TIANGEN BIOTECH CO., LTD. Cat. No. #DP118-02) and sequenced to obtain the TTLEQQYNK TCRs (i.e., TCR010, TCR013, TCR028, and TCR090).

### (2) Packaging of Lentivirus

### Experimental culture medium: 10% FBS (Lonsera, Cat. No. S711-001), DMEM (Cytiva, Cat. No. SH30243.01)

293T cells (the preservation institution is the American Type Culture Collection (ATCC), and the deposit Cat. No. is CRL-1573) were prepared and cultured in a 10 cm dish. Plasmid transfection was initiated when the confluence did not exceed 80%, with a 1:1 ratio of the viral packaging plasmid and TTLEQQYNK TCR plasmid, for a total of 10 µg. The above plasmids were added to serum-free DMEM medium and mixed with PEI (polyethylenimine), and then the mixed solution with plasmids was added to the 293T cells and cultured at 37°C. After 72 hours, the cell supernatant was concentrated using a 100 kd ultrafiltration tube to collect the viral vector.

### Example 3 Construction and functional identification of Jurkat cell line expressing TCR specific for the TTLEQQYNK antigenic short peptide

### Expression method of nuclear factor of activated T cells (NFAT) reporter gene

The following experiments were performed to demonstrate the specific activation response of TCR-transduced T cells to target cells. Flow cytometry was used to detect NFAT expression levels as readout values for T cell activation.

### (1) Reagents

Experimental culture medium: 10% FBS (Lonsera, Cat. No. S711-001), RPMI1640 (ThermoFisher, Cat. No. C11875500BT)

### (2) Methods

### Preparation of target cells

The target cells used in this experiment were T2-A11 cells (T2 cells were deposited in ATCC with a deposit Cat. No. of CRL-1992. T2-A11 cells were constructed based on T2 cells with reference to Cancer Biology & Therapy, 8:21, 2025-2032). Target cells were prepared in experimental culture medium, the concentration of the target cells were adjusted to 1.6×10⁶ cells/ml, and 50 µl was taken from each well to obtain 80,000 cells/well.

### Preparation of effector cells

The effector cells of this experiment were Jurkat-CD8-NFAT (JK8NF) cells transduced with the TCR of the present application, and JK8NF cells not transfected with the TCR of the present application were used as the control group.

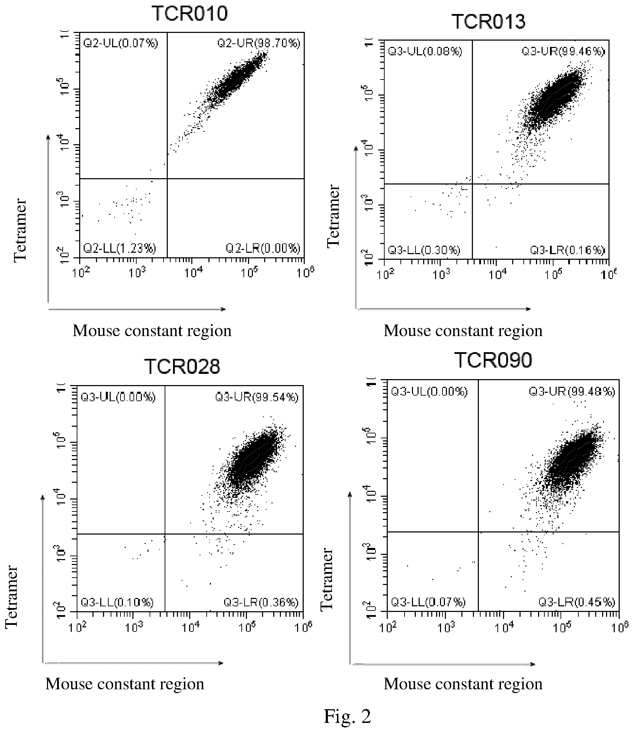

The lentivirus carrying the TCR gene of the present application obtained in Example 2 were added into the JK8NF cells (Jurkat cells were deposited in ATCC with the deposit Cat. No. TIB-152. JK8NF cells were constructed based on Jurkat cells with reference to Cancer Res 2006; 66(23): 11455-61, Front. Immunol.11:633) with an MOI (Multiplicity of Infection) of 10; 72 hours later, the positive rate of the transfection was determined by flow cytometry as about 100% (the result is shown in Fig. 2). The concentration of the effector cells after expanded culture was adjusted to 1.6×10⁶ cells/ml, and 50 µl was taken from each well to obtain 80,000 cells/well.

### Preparation of short peptide solution

The short peptide (TTLEQQYNK) was diluted from the original concentration 5 mg/ml to 400 µg/ml, and then diluted sequentially in a 10-fold ratio to 40 µg/ml, 4 µg/ml, 0.4 µg/ml, 0.04 µg/ml, 0.004 µg/ml, and 0.0004 µg/ml.

50 µl was taken from each well to make the final concentration of the short peptide in the 96-well plate 100 µg/ml, 10 µg/ml, 1 µg/ml, 0.1 µg/ml, 0.01 µg/ml, 0.001 µg/ml, and 0.0001 µg/ml, respectively.

Finally, 50 µl of target cells, 50 µl of effector cells, 50 µl of short peptide dilution of corresponding concentration, and 50 ul of culture medium were added to each well of a 96-well flat-bottom plate, and incubated in a cell culture incubator at 37°C for 12 hours.

### (3) Results

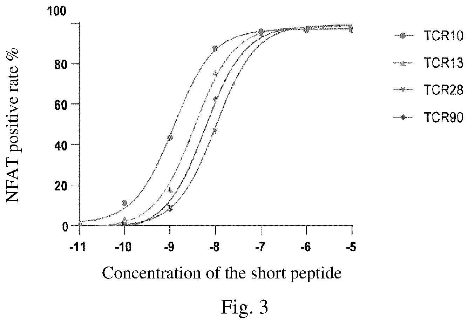

The expression of NFAT in T cells transduced with the TCR of the present application, which respond to target cells loaded with the TTLEQQYNK antigenic short peptide, was examined by the method described above. The expression level curve of NFAT was plotted using Graphpad prism8, and the results are shown in Fig. 3.

As can be seen in Fig. 3, T cells transduced with the TCR of the present application have a good activation response to target cells loaded with their specific short peptides.

### Example 4. Construction and functional identification of primary T cells expressing TCR specific for TTLEQQYNK antigenic short peptide

### Method for real-time monitoring of target cell proliferation

### (1) Reagents

Experimental culture medium: 10% FBS (ThermoFisher, Cat. No. 10099-044), RPMI1640 (ThermoFisher, Cat. No. C11875500BT)

### (2) Methods

### Preparation of effector T cell

The effector cells (T cells) of this experiment were T cells transduced with the TCR of the present application, and T cells of the same volunteer that were not transfected with the TCR of the present application were used as the control group.

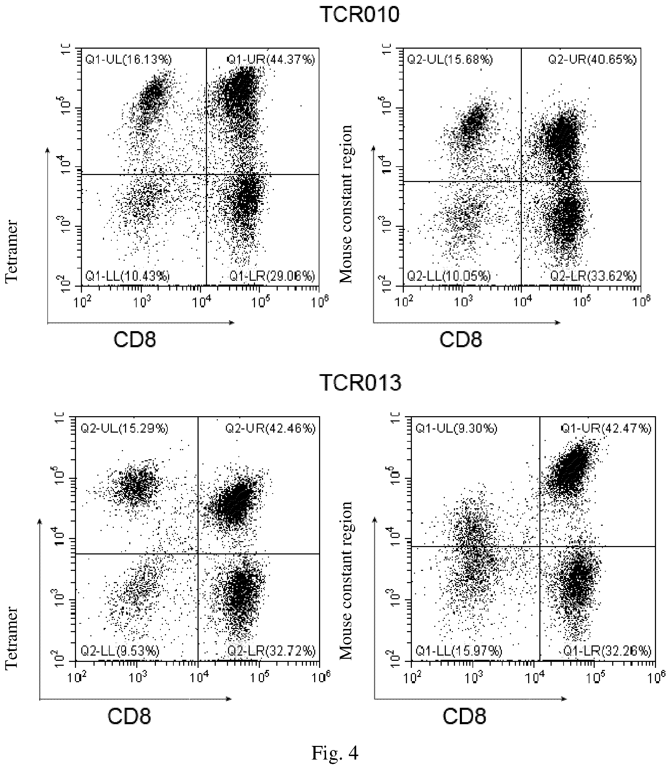

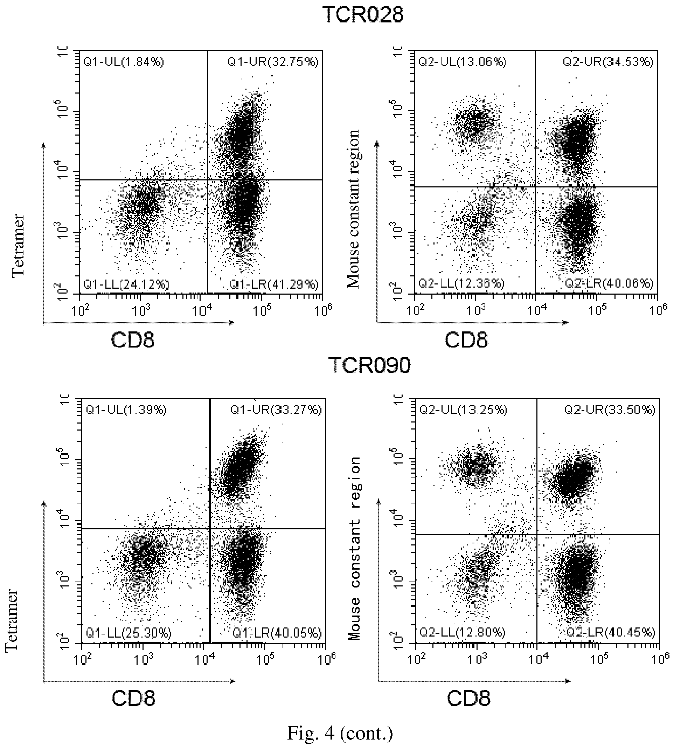

Peripheral blood of the volunteer was centrifuged by density gradient to obtain peripheral blood mononuclear cells; the peripheral blood mononuclear cells were placed in 24-well plates with a concentration of 5.0×10⁵/500µl per well, and totally 1×10⁶ cells were collected. After stimulation with anti-CD3/CD28 magnetic beads, the T cells were cultured in a 37°C, 5% CO₂ incubator. After 24 hours, the cell clustering was observed, and the TCR lentivirus obtained in Example 2 was added for transduction at an MOI (multiplicity of infection) of 2. The cells were amplified in 1640 medium containing 10% FBS and 200 IU/ml IL-2 until 3-4 days after transduction, and the TCR transfection efficiency was identified by flow cytometry (the results are shown in Fig. 4). The concentration of the effector cells after expansion culture was adjusted to 5.0×10⁴ positive cells/ml, and 100 µl was taken from each well to obtain 5000 positive cells/well.

### Preparation of target cells

The target cells used in this experiment were SK-MEL-28-E6. The genotype of SK-MEL-28 (deposited in ATCC) was A*11:01, but it does not express E6 itself. Therefore, SK-MEL-28-E6 cells were constructed as target cells using lentiviral vector overexpressing the E6 gene. The target cells were prepared in experimental culture medium, the target cell concentration was adjusted to 5.0×10⁴ cells/ml, and 100 µl was taken from each well to obtain 5000 cells/well.

Target cell proliferation was monitored by RTCA x Celligence system

100 µl of T cells and 100 µl of the target cells prepared above were added into the E-plate, then the E-plate was installed into a RTCA analyzer, and cultured in a 37°C, 5% CO₂ incubator for 90 h to observe the real-time proliferation curve of the target cells.

### (3) Results

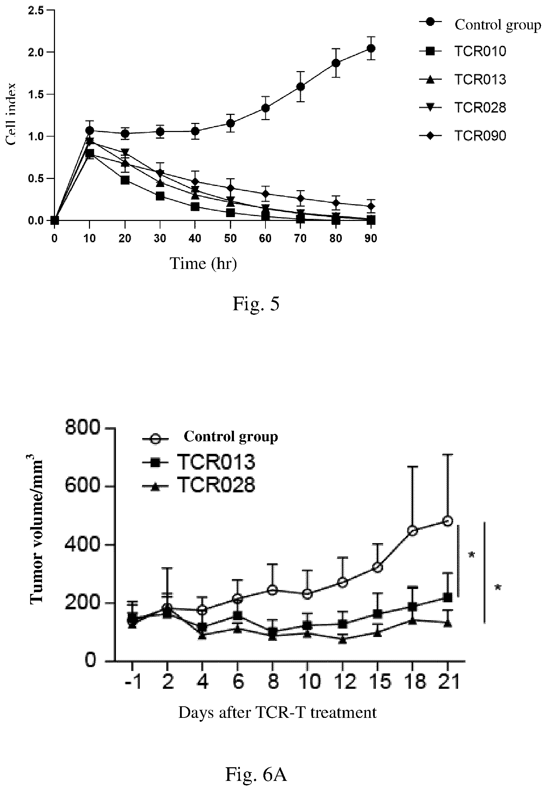

The function of T cells transduced with the TCR of the present application was tested by real-time monitoring of target cell proliferation assay (as described above). Graphpad prism8 was used to plot the cell index (a parameter representing cell number generated by the RTCA x Celligence system) of cell proliferation in each well. The experimental results are shown in Fig. 5.

As can be seen from Fig. 5, T cells expressing TCR specific for the TTLEQQYNK-A*11:01 antigenic short peptide have a strong killing function against E6-expressing cell lines.

### Example 5. Identification of the primary T cells expressing TCR specific for TTLEQQYNK antigenic short peptide for function of inhibiting tumor growth in vivo

The following experiments were performed to demonstrate the inhibitory effect of TCR-transduced T cells on tumor growth. The changes of tumor volumes were observed to evaluate the function of T cells in killing tumors. *In vitro* experiments showed that the four TCRs had similar effects in killing tumors. Therefore, only TCR013 and TCR028 were selected as representatives for identification of their function in inhibiting tumor growth *in vivo.*

### (1) Reagents and mice

Experimental culture medium: 10% FBS (ThermoFisher, Cat. No. 10099-044), RPMI1640 (ThermoFisher, Cat. No. C11875500BT)
NCG mice: purchased from GemPharmatech Co. Ltd., female, 4-6 weeks old, genotypes are (prkdc) ko/ko, (Il2rg female) ko/ko.

### (2) Methods

### Subcutaneous inoculation of tumor cells

The tumor cells used in this experiment are SK-MEL-28-E6. The tumor cells were prepared in physiological saline, the tumor cell concentration was adjusted to 5.0×10⁷ cells/ml, and 200 µl was subcutaneously inoculated into each mouse to obtain 1.0×10⁷ cells/mouse. When the tumor volume grew to about 100 cubic millimeters, the effector T cells were inoculated.

### Preparation of the effector T cells

The effector cells (T cells) of this experiment were T cells transduced with the TCR of the present application, and T cells of the same volunteer that were not transfected with the TCR of the present application were used as the control group. There were 5 mice in each group.

Peripheral blood of the volunteer was centrifuged by density gradient to obtain peripheral blood mononuclear cells; the peripheral blood mononuclear cells were placed in 24-well plates with a concentration of 5.0×10⁵/500µl per well, and totally 1×10⁶ cells were collected. After stimulation with anti-CD3/CD28 magnetic beads, the T cells were cultured in a 37°C, 5% CO₂ incubator. After 24 hours, the cell clustering was observed, and lentivirus comprising the TCR genes (TCR013 and TCR028 genes) obtained in Example 2 were added for transduction at an MOI (multiplicity of infection) of 2. The cells were amplified in 1640 medium containing 10% FBS and 200 IU/ml IL-2 until 3-4 days after transduction, and the TCR transfection efficiency was identified by flow cytometry. The concentration of the effector cells after expansion culture was adjusted to 5.0×10⁷ positive cells/ml, and a dose of 200 µl was injected into each mouse through the ophthalmic vein to obtain 1.0×10⁷ positive cells/mouse.

### (3) Results

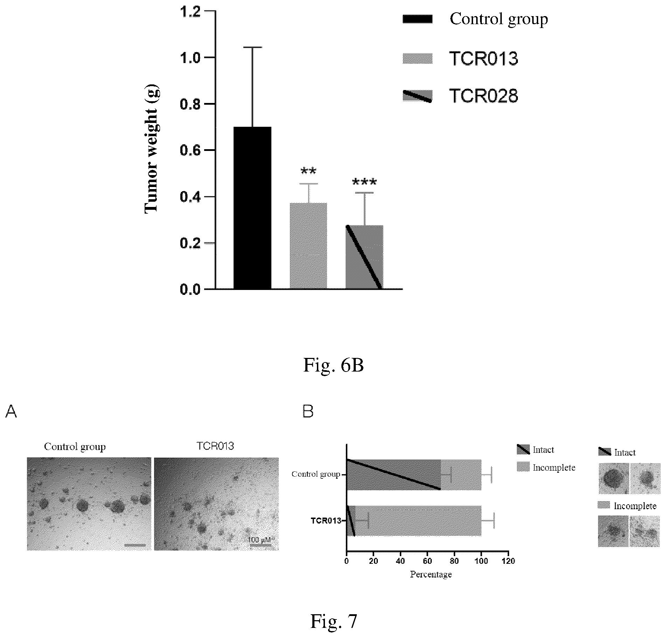

The volume of the tumor after T cell inoculation was observed and recorded, and the effect of the primary T cells expressing the TCR of the present application in treating tumors was evaluated according to the size change in tumor volume. The growth curve of the tumor was plotted using Graphpad prism8, and the experimental results are shown in Figs. 6A and 6B, where Fig. 6A is a schematic diagram of the change in tumor volume over time after T cell inoculation, and Fig. 6B is a bar graph of tumor weight after inoculation of T cells comprising different TCRs.

As can be seen from Figs. 6A and 6B, T cells expressing TCR specific for the TTLEQQYNK-A*11:01 antigenic short peptide can effectively inhibit the growth of E6-positive tumors.

### Example 6. Killing function of T cells expressing TCR specific for TTLEQQYNK-A*11:01 antigenic short peptide on primary HPV tumors

### (1) Reagents

Experimental culture medium: OrganoPro Cervical Cancer Organoid Culture Medium Kit (K2 ONCOLOGY, Cat. No. K2O-M-CC)

### (2) Methods

8,000 HPV16⁺HLA-A*11:01⁺ cervical cancer organoids KOCC-002S4 (K2 Oncology) were co-cultured with 8,000 primary T cells transduced with TCR013 (TCR013) or control T cells not transduced with TCR (control group). 72 hours later, the growth status of the organoids was observed under a microscope and photographed.

### (3) Results

The growth status of the organoids was observed through a microscope, the typical field of vision was photographed, and the number of morphologically complete and incomplete organoids in the fields of vision were counted. The results are shown in Figs. 7A to 7B, wherein Fig. 7A shows the growth status of the organoids observed through the microscope, and Fig. 7B is a statistical diagram of the number of morphologically complete and incomplete organoids.

As can be seen from Figs. 7A to 7B, T cells expressing TCR specific for TTLEQQYNK-A*11:01 antigenic short peptide can significantly kill primary cervical cancer organoid cells.

In summary, the T cells transduced with the TCR in the present application can be specifically activated and have a strong killing effect on tumor cells expressing A1101 and HPV.

The T cells have a strong killing effect on the cell lines expressing E6 and can effectively inhibit the growth of E6-positive tumors.

The above are only preferred embodiments of the present application and do not constitute any other form of limitation to the present application. Any people skilled in the art may use the technical contents disclosed above to change or modify them into equivalent embodiments with equivalent changes. However, any simple modifications, equivalent changes and modifications made to the above embodiments based on the essential technical content of the present application, without departing from the content of the technical solution of the present application, still fall within the protection scope of the technical solution of the present application.

## Claims

1. Use of an antigenic short peptide in screening drugs for treating HPV-related diseases, wherein the amino acid sequence of the antigenic short peptide is represented by SEQ ID NO: 1 or SEQ ID NO: 2.

2. The use according to claim 1, wherein the HPV-related disease is chronic HPV infection, cervical intraepithelial neoplasia, cervical cancer, head and neck cancer, anal cancer, penile cancer, vaginal cancer or vulvar cancer.

3. Use of an antigenic short peptide in screening drugs for treating chronic HPV infection, cervical intraepithelial neoplasia, cervical cancer, head and neck cancer, anal cancer, penile cancer, vaginal cancer, or vulvar cancer, wherein the amino acid sequence of the antigenic short peptide is represented by SEQ ID NO: 1 or SEQ ID NO: 2.

4. The use according to any one of claims 1-3, wherein the drug is a T cell receptor (TCR) for binding to an antigenic short peptide-HLA-A1101 complex comprising the antigenic short peptide.

5. A T cell receptor (TCR), wherein the TCR comprises an α chain comprising a variable region and/or a β chain comprising a variable region, and the variable region of the α chain comprises:
a complementarity determining region 1 (CDR1) having an amino acid sequence of SEQ ID NO: 3 or SEQ ID NO: 9; and/or
a complementarity determining region 2 (CDR2) having an amino acid sequence of SEQ ID NO:4 or SEQ ID NO:10.

6. The T cell receptor (TCR) according to claim 5, wherein the variable region of the β chain comprises:
a complementarity determining region 1 (CDR1) having an amino acid sequence of SEQ ID NO: 6; and/or
a complementarity determining region 2 (CDR2) having an amino acid sequence of SEQ ID NO:7.

7. The T cell receptor (TCR) according to claim 5 or 6, wherein the variable region of the α chain comprises: a complementarity determining region 3 (CDR3) comprising an amino acid sequence of SEQ ID NO: 5, SEQ ID NO: 11, SEQ ID NO: 13 or SEQ ID NO: 15; and/or
the variable region of the β chain comprises: a complementarity determining region 3 (CDR3) comprising an amino acid sequence of SEQ ID NO:8, SEQ ID NO:12, SEQ ID NO:14 or SEQ ID NO: 16;
preferably, the variable region of the α chain further comprises a first leader sequence; and/or
the variable region of the β chain further comprises a second leader sequence;
preferably, the amino acid sequence of the variable region of the α chain is represented by SEQ ID NO: 20, SEQ ID NO: 22, SEQ ID NO: 24 or SEQ ID NO: 26, or is an amino acid sequence having at least 90% sequence identity with SEQ ID NO: 20, SEQ ID NO: 22, SEQ ID NO: 24 or SEQ ID NO: 26; and/or
the amino acid sequence of the variable region of the β chain is represented by SEQ ID NO:21, SEQ ID NO:23, SEQ ID NO:25 or SEQ ID NO:27, or is an amino acid sequence having at least 90% sequence identity with SEQ ID NO:21, SEQ ID NO:23, SEQ ID NO:25 or SEQ ID NO:27;
preferably, the α chain further comprises an α constant region, and/or the β chain further comprises a β constant region; preferably, the constant region is a mouse constant region or a human constant region.

8. The T cell receptor (TCR) according to any one of claims 5-7, wherein the TCR is isolated or purified, or is recombinant;
preferably, the TCR is human;
preferably, the TCR is monoclonal;
preferably, the TCR is a single chain;
preferably, the TCR comprises two chains;
preferably, the TCR is in a cell-bound form or in a soluble form, preferably in a soluble form;
preferably, the TCR binds to the antigenic short peptide-HLAA1101 complex, and preferably, the amino acid sequence of the antigenic short peptide is represented by SEQ ID NO: 1 or SEQ ID NO: 2.

9. A nucleic acid molecule, wherein the nucleic acid molecule comprises: a nucleotide sequence encoding the TCR or the α chain or β chain thereof according to any one of claims 5-8.

10. The nucleic acid molecule according to claim 9, wherein the nucleotide sequence encoding the α chain comprises a nucleotide sequence of SEQ ID NO: 30, SEQ ID NO: 32, SEQ ID NO: 34 or SEQ ID NO: 36; and/or
the nucleotide sequence encoding the β chain comprises a nucleotide sequence of SEQ ID NO:31, SEQ ID NO:33, SEQ ID NO:35 or SEQ ID NO:37.

11. A vector, wherein the vector comprises the nucleic acid molecule according to claim 9 or 10.

12. The vector according to claim 11, wherein the vector is an expression vector;
preferably, the vector is a viral vector, preferably a retroviral vector;
preferably, the viral vector is a lentiviral vector.

13. An engineered cell, comprising: the TCR according to any one of claims 5-8, the nucleic acid molecule according to any one of claims 9-10, or the vector according to any one of claims 11-12.

14. The engineered cell according to claim 13, wherein the TCR is heterologous to the cell;
preferably, the engineered cell is a cell line;
preferably, the engineered cell is a primary cell obtained from a subject; preferably, the subject is a mammalian subject, preferably a human;
preferably, the engineered cell is a T cell or a NK cell; preferably, the T cell is a T cell isolated from peripheral blood;
preferably, the T cell is CD8+ or CD4+.

15. A method for producing the engineered cell according to any one of claims 13-14, comprising introducing the nucleic acid molecule according to any one of claims 9-10 or the vector according to any one of claims 11-12 into a cell in vitro or ex vivo.

16. The method according to claim 15, wherein the vector is a viral vector, and the introduction is performed by transduction.

17. A pharmaceutical composition, comprising: the T cell receptor (TCR) according to any one of claims 5-8, the nucleic acid molecule according to any one of claims 9-10, the vector according to any one of claims 11-12, or the engineered cell according to any one of claims 13-14;
preferably, the pharmaceutical composition further comprises a pharmaceutically acceptable carrier or adjuvant.

18. Use of the T cell receptor (TCR) according to any one of claims 5-8, the nucleic acid molecule according to any one of claims 9-10, the vector according to any one of claims 11-12, the engineered cell according to any one of claims 13-14, or the pharmaceutical composition according to claim 17 in the preparation of a medicament for treating HPV-related diseases;
preferably, the HPV-related disease is chronic HPV infection, cervical intraepithelial neoplasia, cervical cancer, head and neck cancer, anal cancer, penile cancer, vaginal cancer or vulvar cancer.

19. A method for treating HPV-related diseases, comprising administering the T cell receptor (TCR) according to any one of claims 5-8, the nucleic acid molecule according to any one of claims 9-10, the vector according to any one of claims 11-12, the engineered cell according to any one of claims 13-14, or the pharmaceutical composition according to claim 17 to a subject in need thereof.

20. The method according to claim 19, wherein the HPV-related disease is chronic HPV infection, cervical intraepithelial neoplasia, cervical cancer, head and neck cancer, anal cancer, penile cancer, vaginal cancer or vulvar cancer.
